(19) Europäisches Patentamt
European Patent Office
Office européen des brevets

(11) EP 2 757 379 A1

(12) EUROPEAN PATENT APPLICATION

(43) Date of publication:
**23.07.2014 Bulletin 2014/30**

(51) Int Cl.:
***G01N 33/68*** *(2006.01)*

(21) Application number: **13151538.9**

(22) Date of filing: **16.01.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.
80539 München (DE)**

(72) Inventors:
- **Hartmann, Marcus D.
72076 Tübingen (DE)**
- **Hernandez-Alvarez, Birte
72135 Dettenhausen (DE)**
- **Lupas, Andrei N.
72076 Tübingen (DE)**

(74) Representative: **Arth, Hans-Lothar
ABK Patent Attorneys
Jasminweg 9
14052 Berlin (DE)**

# (54) Method to screen for the teratogenic potential of substances

(57) The present invention relates to co-crystal forms of a bacterial cereblon homologue together with thalidomide, crystal structure information obtained from them, methods of preparing such co-crystal forms. The invention refers further to an *in vitro* method to analyze the teratogenic potential of substances, preferably pharmaceutically active substances, via their binding to the protein cereblon, a cellular target of thalidomide, and refers to the substrate 1-(N-2-MANT-imidoethyl)-uracil as well as a mutant bacterial cereblon homologue from *Magnetospirillum gryphiswaldense* both compounds of the inventive method.

**Figure 5**

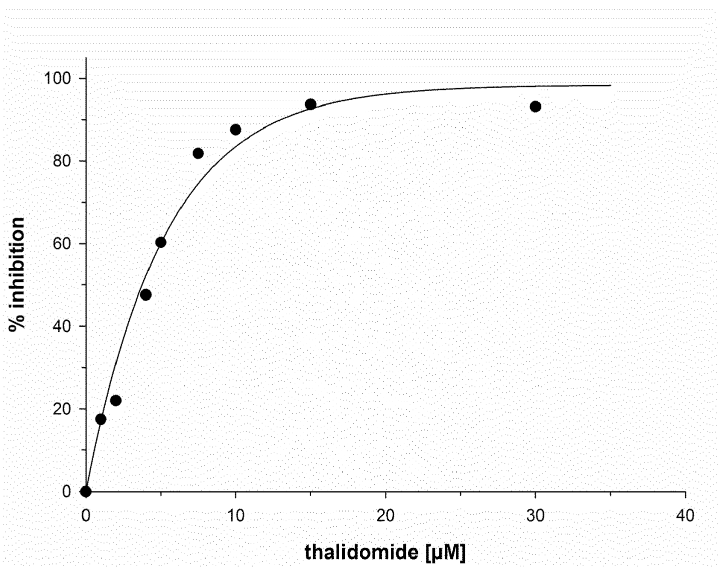


EP 2 757 379 A1

**SEQUENCE LISTING**

SEQUENCE LISTING

<110> Max-Planck-Gesellschaft zur Förderung der Wissenschaften

<120> Method to screen for the teratogenic potential of substances

<130> GAR-P03540EP

<160> 15

<170> PatentIn version 3.5

<210> 1
<211> 124
<212> PRT
<213> Magnetospirillum gryphiswaldense

<400> 1

Met Pro Leu Asp Ala Gly Gly Gln Asn Ser Thr Gln Met Val Leu Ala
Pro Gly Ala Ser Ile Phe Arg Cys Arg Gln Cys Gly Gln Thr Ile Ser
Arg Arg Asp Trp Leu Leu Pro Met Gly Gly Asp His Glu His Val Val
Phe Asn Pro Ala Gly Met Ile Phe Arg Val Trp Cys Phe Ser Leu Ala
Gln Gly Leu Arg Leu Ile Gly Ala Pro Ser Gly Glu Phe Ser Trp Phe
Lys Gly Tyr Asp Trp Thr Ile Ala Leu Cys Gly Gln Cys Gly Ser His
Leu Gly Trp His Tyr Glu Gly Gly Ser Gln Pro Gln Thr Phe Phe Gly
Leu Ile Lys Asp Arg Leu Ala Glu Gly Pro Ala Asp

<210> 2
<211> 375
<212> DNA
<213> Magnetospirillum gryphiswaldense

<400> 2
atgcccctgg atgccggggg acaaaacagc acccagatgg tgctggcccc cggcgccagc 60
atctttcgct gccgccagtg cgggcaaacc atcagccgcc gcgactggct gctgcctatg 120
ggcggcgacc atgaacacgt ggtgttcaat ccggcgggga tgatttttcg cgtctggtgt 180
ttttccctgg cgcagggcct gcgcctgatc ggcgcgcctt cgggggaatt tagctggttc 240
aagggctatg actggaccat cgccctgtgc ggccaatgcg gcagccatct gggctggcat 300
tatgaaggcg gcagccaacc gcaaactttc ttcggtctga tcaaggatcg gttggccgag 360
ggaccggcgg attag 375

<210> 3
<211> 127
<212> PRT
<213> Magnetospirillum gryphiswaldense

<400> 3

Gly Ala Met Gly Pro Leu Asp Ala Gly Gly Gln Asn Ser Thr Gln Met
Val Leu Ala Pro Gly Ala Ser Ile Phe Arg Cys Arg Gln Cys Gly Gln
Thr Ile Ser Arg Arg Asp Trp Leu Leu Pro Met Gly Gly Asp His Glu
His Val Val Phe Asn Pro Ala Gly Met Ile Phe Arg Val Trp Cys Phe
Ser Leu Ala Gln Gly Leu Arg Leu Ile Gly Ala Pro Ser Gly Glu Phe
Ser Trp Phe Lys Gly Tyr Asp Trp Thr Ile Ala Leu Cys Gly Gln Cys
Gly Ser His Leu Gly Trp His Tyr Glu Gly Gly Ser Gln Pro Gln Thr
Phe Phe Gly Leu Ile Lys Asp Arg Leu Ala Glu Gly Pro Ala Asp

<210> 4
<211> 378
<212> DNA
<213> Artificial

<220>
<223> codon optomized sequence for E. coli

<400> 4
atgggtccgc tggatgcagg tggtcagaac tcaacgcaaa tggtcctggc tccgggtgct 60
tcgatcttcc gctgtcgtca atgtggtcag accattagcc gtcgcgattg gctgctgccg 120
atgggcggtg accatgaaca cgtggttttt aacccggcag gcatgatttt tcgtgtctgg 180
tgcttcagcc tggcacaggg tctgcgtctg atcggtgcac cgagcggtga attttcttgg 240
ttcaaaggct atgattggac gatcgcgctg tgcggccaat gtggtagtca tctgggttgg 300
cactacgaag gcggttccca accgcagacg tttttcggcc tgattaaaga ccgcctggca 360
gaaggtccgg ctgactga 378

<210> 5
<211> 378
<212> DNA
<213> Artificial

<220>
<223> W36/59F mutant

<400> 5
atgggtccgc tggatgcagg tggtcagaac tcaacgcaaa tggtcctggc tccgggtgct 60
tcgatcttcc gctgtcgtca atgtggtcag accattagcc gtcgcgattt cctgctgccg 120
atgggcggtg accatgaaca cgtggttttt aacccggcag gcatgatttt tcgtgtcttc 180
tgcttcagcc tggcacaggg tctgcgtctg atcggtgcac cgagcggtga attttcttgg 240
ttcaaaggct atgattggac gatcgcgctg tgcggccaat gtggtagtca tctgggttgg 300
cactacgaag gcggttccca accgcagacg tttttcggcc tgattaaaga ccgcctggca 360

gaaggtccgg ctgactga 378

<210> 6
<211> 127
<212> PRT
<213> Artificial

<220>
<223> W36/59F Mutant

<400> 6

Gly Ala Met Gly Pro Leu Asp Ala Gly Gly Gln Asn Ser Thr Gln Met
Val Leu Ala Pro Gly Ala Ser Ile Phe Arg Cys Arg Gln Cys Gly Gln
Thr Ile Ser Arg Arg Asp Phe Leu Leu Pro Met Gly Gly Asp His Glu
His Val Val Phe Asn Pro Ala Gly Met Ile Phe Arg Val Phe Cys Phe
Ser Leu Ala Gln Gly Leu Arg Leu Ile Gly Ala Pro Ser Gly Glu Phe
Ser Trp Phe Lys Gly Tyr Asp Trp Thr Ile Ala Leu Cys Gly Gln Cys
Gly Ser His Leu Gly Trp His Tyr Glu Gly Gly Ser Gln Pro Gln Thr
Phe Phe Gly Leu Ile Lys Asp Arg Leu Ala Glu Gly Pro Ala Asp

<210> 7
<211> 125
<212> PRT
<213> Homo sapiens

<400> 7

Cys Thr Ser Leu Cys Cys Lys Gln Cys Gln Glu Thr Glu Ile Thr Thr
Lys Asn Glu Ile Phe Ser Leu Ser Leu Cys Gly Pro Met Ala Ala Tyr
Val Asn Pro His Gly Tyr Val His Glu Thr Leu Thr Val Tyr Lys Ala
Cys Asn Leu Asn Leu Ile Gly Arg Pro Ser Thr Glu His Ser Trp Phe
Pro Gly Tyr Ala Trp Thr Val Ala Gln Cys Lys Ile Cys Ala Ser His
Ile Gly Trp Lys Phe Thr Ala Thr Lys Lys Asp Met Ser Pro Gln Lys
Phe Trp Gly Leu Thr Arg Ser Ala Leu Leu Pro Thr Ile Pro Asp Thr
Glu Asp Glu Leu Ser Pro Asp Arg Val Ile Leu Cys Leu

<210> 8
<211> 107
<212> PRT
<213> Magnetospirillum gryphiswaldense

<400> 8

Gly Ala Ser Ile Phe Arg Cys Arg Gln Cys Gly Gln Thr Ile Ser Arg
Arg Asp Trp Leu Leu Pro Met Gly Gly Asp His Glu His Val Val Phe
Asn Pro Ala Gly Met Ile Phe Arg Val Trp Cys Phe Ser Leu Ala Gln

Gly Leu Arg Leu Ile Gly Ala Pro Ser Gly Glu Phe Ser Trp Phe Lys
Gly Tyr Asp Trp Thr Ile Ala Leu Cys Gly Gln Cys Gly Ser His Leu
Gly Trp His Tyr Glu Gly Gly Ser Gln Pro Gln Thr Phe Phe Gly Leu
Ile Lys Asp Arg Leu Ala Glu Gly Pro Ala Asp

<210> 9
<211> 149
<212> PRT
<213> Caenorhabditis elegans

<400> 9

Met Gly Arg His Val Gly Glu Leu Leu Cys Arg Gln Cys Gly Ala Ser
Ile Thr Arg Gln Ser Glu Leu Ile Asn Ile Thr Gly Val Asp Gln Ser
Gln Leu Gln Tyr Glu Tyr Asp Phe Pro Leu Ala Gly Lys Thr Thr Lys
Val Asn Val Leu Thr Asn Pro Glu Asn Gln Lys Phe His Val Phe Gly
Ala Lys Thr Ala His Leu His Phe His Gly Thr Pro Gln Ser His Ala
Thr Trp Tyr Pro Gly Tyr Lys Trp Thr Ile Cys Leu Cys Lys Ser Cys
Ser Arg His Met Gly Trp Tyr Phe Glu Pro Glu Lys Ser Thr Ala Val
Ser Glu Lys Lys Ser Phe Val Gly Leu Val Leu Asp Asn Val Ile Ser
Ala Asp Tyr Val Asp Thr Leu Thr Lys Val Pro Asp Phe Lys Gly Glu
Asn Leu Tyr Phe Gln

<210> 10
<211> 40
<212> DNA
<213> Artificial

<220>
<223> primer W36F se

<400> 10
ccattagccg tcgcgatttc ctgctgccga tgggcggtga 40

<210> 11
<211> 40
<212> DNA
<213> Artificial

<220>
<223> Primer W36F as

<400> 11
tcaccgccca tcggcagcag gaaatcgcga cggctaatgg 40

<210> 12
<211> 39
<212> DNA
<213> Artificial

<220>
<223> Primer W59F se

<400> 12
ggcatgattt ttcgtgtctt ctgcttcagc ctggcacag 39

<210> 13
<211> 39
<212> DNA
<213> Artificial

<220>
<223> primer W59 as

<400> 13
ctgtgccagg ctgaagcaga agacacgaaa aatcatgcc 39

<210> 14
<211> 38
<212> DNA
<213> Artificial Sequence

<220>
<223> Primer Y101F se

<400> 14
gtcatctggg ttggcacttc gaaggcggtt cccaaccg 38

<210> 15
<211> 38
<212> DNA
<213> Artificial Sequence

<220>
<223> Primer Y101F as

<400> 15
cggttgggaa ccgccttcga agtgccaacc cagatgac 38

## SEQUENCE LISTING

SEQUENCE LISTING

<110> Max-Planck-Gesellschaft zur Förderung der Wissenschaften

<120> Method to screen for the teratogenic potential of substances

<130> GAR-P03540EP

<160> 15

<170> PatentIn version 3.5

<210> 1
<211> 124
<212> PRT
<213> Magnetospirillum gryphiswaldense

<400> 1

Met Pro Leu Asp Ala Gly Gly Gln Asn Ser Thr Gln Met Val Leu Ala
Pro Gly Ala Ser Ile Phe Arg Cys Arg Gln Cys Gly Gln Thr Ile Ser
Arg Arg Asp Trp Leu Leu Pro Met Gly Gly Asp His Glu His Val Val
Phe Asn Pro Ala Gly Met Ile Phe Arg Val Trp Cys Phe Ser Leu Ala
Gln Gly Leu Arg Leu Ile Gly Ala Pro Ser Gly Glu Phe Ser Trp Phe
Lys Gly Tyr Asp Trp Thr Ile Ala Leu Cys Gly Gln Cys Gly Ser His
Leu Gly Trp His Tyr Glu Gly Gly Ser Gln Pro Gln Thr Phe Phe Gly
Leu Ile Lys Asp Arg Leu Ala Glu Gly Pro Ala Asp

<210> 2
<211> 375
<212> DNA
<213> Magnetospirillum gryphiswaldense

<400> 2
atgcccctgg atgccggggg acaaaacagc acccagatgg tgctggcccc cggcgccagc 60
atctttcgct gccgccagtg cgggcaaacc atcagccgcc gcgactggct gctgcctatg 120
ggcggcgacc atgaacacgt ggtgttcaat ccggcgggga tgatttttcg cgtctggtgt 180
ttttccctgg cgcagggcct gcgcctgatc ggcgcgcctt cgggggaatt tagctggttc 240
aagggctatg actggaccat cgccctgtgc ggccaatgcg gcagccatct gggctggcat 300
tatgaaggcg gcagccaacc gcaaactttc ttcggtctga tcaaggatcg gttggccgag 360
ggaccggcgg attag 375

<210> 3
<211> 127
<212> PRT
<213> Magnetospirillum gryphiswaldense

<400> 3

Gly Ala Met Gly Pro Leu Asp Ala Gly Gly Gln Asn Ser Thr Gln Met
Val Leu Ala Pro Gly Ala Ser Ile Phe Arg Cys Arg Gln Cys Gly Gln
Thr Ile Ser Arg Arg Asp Trp Leu Leu Pro Met Gly Gly Asp His Glu
His Val Val Phe Asn Pro Ala Gly Met Ile Phe Arg Val Trp Cys Phe
Ser Leu Ala Gln Gly Leu Arg Leu Ile Gly Ala Pro Ser Gly Glu Phe
Ser Trp Phe Lys Gly Tyr Asp Trp Thr Ile Ala Leu Cys Gly Gln Cys
Gly Ser His Leu Gly Trp His Tyr Glu Gly Gly Ser Gln Pro Gln Thr
Phe Phe Gly Leu Ile Lys Asp Arg Leu Ala Glu Gly Pro Ala Asp

<210> 4
<211> 378
<212> DNA
<213> Artificial

<220>
<223> codon optomized sequence for E. coli

<400> 4
atgggtccgc tggatgcagg tggtcagaac tcaacgcaaa tggtcctggc tccgggtgct 60
tcgatcttcc gctgtcgtca atgtggtcag accattagcc gtcgcgattg gctgctgccg 120
atgggcggtg accatgaaca cgtggttttt aacccggcag gcatgatttt tcgtgtctgg 180
tgcttcagcc tggcacaggg tctgcgtctg atcggtgcac cgagcggtga attttcttgg 240
ttcaaaggct atgattggac gatcgcgctg tgcggccaat gtggtagtca tctgggttgg 300
cactacgaag gcggttccca accgcagacg tttttcggcc tgattaaaga ccgcctggca 360
gaaggtccgg ctgactga 378

<210> 5
<211> 378
<212> DNA
<213> Artificial

<220>
<223> W36/59F mutant

<400> 5
atgggtccgc tggatgcagg tggtcagaac tcaacgcaaa tggtcctggc tccgggtgct 60
tcgatcttcc gctgtcgtca atgtggtcag accattagcc gtcgcgattt cctgctgccg 120
atgggcggtg accatgaaca cgtggttttt aacccggcag gcatgatttt tcgtgtcttc 180
tgcttcagcc tggcacaggg tctgcgtctg atcggtgcac cgagcggtga attttcttgg 240
ttcaaaggct atgattggac gatcgcgctg tgcggccaat gtggtagtca tctgggttgg 300
cactacgaag gcggttccca accgcagacg tttttcggcc tgattaaaga ccgcctggca 360

gaaggtccgg ctgactga 378

<210> 6
<211> 127
<212> PRT
<213> Artificial

<220>
<223> W36/59F Mutant

<400> 6

Gly Ala Met Gly Pro Leu Asp Ala Gly Gly Gln Asn Ser Thr Gln Met
Val Leu Ala Pro Gly Ala Ser Ile Phe Arg Cys Arg Gln Cys Gly Gln
Thr Ile Ser Arg Arg Asp Phe Leu Leu Pro Met Gly Gly Asp His Glu
His Val Val Phe Asn Pro Ala Gly Met Ile Phe Arg Val Phe Cys Phe
Ser Leu Ala Gln Gly Leu Arg Leu Ile Gly Ala Pro Ser Gly Glu Phe
Ser Trp Phe Lys Gly Tyr Asp Trp Thr Ile Ala Leu Cys Gly Gln Cys
Gly Ser His Leu Gly Trp His Tyr Glu Gly Gly Ser Gln Pro Gln Thr
Phe Phe Gly Leu Ile Lys Asp Arg Leu Ala Glu Gly Pro Ala Asp

<210> 7
<211> 125
<212> PRT
<213> Homo sapiens

<400> 7

Cys Thr Ser Leu Cys Cys Lys Gln Cys Gln Glu Thr Glu Ile Thr Thr
Lys Asn Glu Ile Phe Ser Leu Ser Leu Cys Gly Pro Met Ala Ala Tyr
Val Asn Pro His Gly Tyr Val His Glu Thr Leu Thr Val Tyr Lys Ala
Cys Asn Leu Asn Leu Ile Gly Arg Pro Ser Thr Glu His Ser Trp Phe
Pro Gly Tyr Ala Trp Thr Val Ala Gln Cys Lys Ile Cys Ala Ser His
Ile Gly Trp Lys Phe Thr Ala Thr Lys Lys Asp Met Ser Pro Gln Lys
Phe Trp Gly Leu Thr Arg Ser Ala Leu Leu Pro Thr Ile Pro Asp Thr
Glu Asp Glu Leu Ser Pro Asp Arg Val Ile Leu Cys Leu

<210> 8
<211> 107
<212> PRT
<213> Magnetospirillum gryphiswaldense

<400> 8

Gly Ala Ser Ile Phe Arg Cys Arg Gln Cys Gly Gln Thr Ile Ser Arg
Arg Asp Trp Leu Leu Pro Met Gly Gly Asp His Glu His Val Val Phe
Asn Pro Ala Gly Met Ile Phe Arg Val Trp Cys Phe Ser Leu Ala Gln

Gly Leu Arg Leu Ile Gly Ala Pro Ser Gly Glu Phe Ser Trp Phe Lys
Gly Tyr Asp Trp Thr Ile Ala Leu Cys Gly Gln Cys Gly Ser His Leu
Gly Trp His Tyr Glu Gly Gly Ser Gln Pro Gln Thr Phe Phe Gly Leu
Ile Lys Asp Arg Leu Ala Glu Gly Pro Ala Asp

<210> 9
<211> 149
<212> PRT
<213> Caenorhabditis elegans

<400> 9

Met Gly Arg His Val Gly Glu Leu Leu Cys Arg Gln Cys Gly Ala Ser
Ile Thr Arg Gln Ser Glu Leu Ile Asn Ile Thr Gly Val Asp Gln Ser
Gln Leu Gln Tyr Glu Tyr Asp Phe Pro Leu Ala Gly Lys Thr Thr Lys
Val Asn Val Leu Thr Asn Pro Glu Asn Gln Lys Phe His Val Phe Gly
Ala Lys Thr Ala His Leu His Phe His Gly Thr Pro Gln Ser His Ala
Thr Trp Tyr Pro Gly Tyr Lys Trp Thr Ile Cys Leu Cys Lys Ser Cys
Ser Arg His Met Gly Trp Tyr Phe Glu Pro Glu Lys Ser Thr Ala Val
Ser Glu Lys Lys Ser Phe Val Gly Leu Val Leu Asp Asn Val Ile Ser
Ala Asp Tyr Val Asp Thr Leu Thr Lys Val Pro Asp Phe Lys Gly Glu
Asn Leu Tyr Phe Gln

<210> 10
<211> 40
<212> DNA
<213> Artificial

<220>
<223> primer W36F se

<400> 10
ccattagccg tcgcgatttc ctgctgccga tgggcggtga 40

<210> 11
<211> 40
<212> DNA
<213> Artificial

<220>
<223> Primer W36F as

<400> 11
tcaccgccca tcggcagcag gaaatcgcga cggctaatgg 40

<210> 12
<211> 39
<212> DNA
<213> Artificial

<220>
<223> Primer W59F se

<400> 12
ggcatgattt ttcgtgtctt ctgcttcagc ctggcacag 39

<210> 13
<211> 39
<212> DNA
<213> Artificial

<220>
<223> primer W59 as

<400> 13
ctgtgccagg ctgaagcaga agacacgaaa aatcatgcc 39

<210> 14
<211> 38
<212> DNA
<213> Artificial Sequence

<220>
<223> Primer Y101F se

<400> 14
gtcatctggg ttggcacttc gaaggcggtt cccaaccg 38

<210> 15
<211> 38
<212> DNA
<213> Artificial Sequence

<220>
<223> Primer Y101F as

<400> 15
cggttgggaa ccgccttcga agtgccaacc cagatgac 38

## Description

The present invention relates to co-crystal forms of a bacterial cereblon homologue together with a ligand like thalidomide, crystal structure information obtained from them and methods of preparing such co-crystal forms. The invention refers further to an in vitro method to analyze the teratogenic potential of substances, preferably pharmaceutically active substances, via their binding to the protein cereblon, a cellular target of thalidomide, and refers to the substrate 1-(N-2-MANT-imidoethyl)-uracil as well as a mutant bacterial cereblon homologue from *Magnetospirillum gryphiswaldense,* both compounds of the inventive method.

A teratogen is an agent that can disturb the development of an embryo or fetus. Classes of teratogens include radiation, maternal infections, chemicals, and drugs. Meanwhile, the legislative authority uses the term toxic for reproduction and instead of CMT substances (carcinogenic, mutagenic, teratogenic) today is spoken of CMR (carcinogenic, mutagenic, toxic for reproduction). The term toxic for reproduction refers to two effects of toxic agents: impairment of fertility and harmful to the unborn child. Nevertheless the assay of the present invention is suitable to screen for substances being harmful to the unborn. Therefore the term "teratogen" as used herein refers to an agent that can disturb the development of an embryo or fetus.

Approximately 10% of human malformations observed in the first year of life are caused by teratogenic, environmental agents, which have several characteristics in common. The susceptibility of the embryo as well as the degree of the adverse effect caused by the agent depends on the stage of development at which the exposure occurred. There exists a quantitative correlation between the magnitude of embryopathic effects and the dose of the teratogenic agent, such that the higher the dose, the more severe is the teratogenic effect. In case of extrapolation of data between different species, a threshold effect and differences due to genetic variability caused by differences in the placental transport, metabolism, absorption and distribution of an agent in mammals and these differences must be taken into account.

One example is thalidomide (marketed under the trademark Contergan), which was first synthesized in 1954 and marketed in 1956 in Germany. It was studied in rodents for a variety of toxicities, and even when administered at very high doses, no side effects were observed. Thalidomide was known in the mid-50s as a sedative. The administration of thalidomide, which at this time was deemed to be the safest sedative, for treatment of morning sickness in the first trimester induced the well-known, serious malformations of limbs, sense organs but also internal organs and the central nervous system of newborns. Worldwide, more than 10,000 children were affected.

Thalidomide ($\alpha$-phthalimido glutarimide) has a chiral character and exist as S- and R-enantiomer, the latter being the sedative wherein the S-enatiomer has teratogenic properties. Both forms may racemize spontaneously *in vivo.* Despite its serious side effects thalidomide was used for the treatment of leprosy and for immunosuppression after transplantation because of its anti-inflammatory and immunomodulatory properties. Thalidomide has also anti-angiogenic and thus also anti-tumor properties. On the basis of these diverse effects in the mid-90s, the pharmaceutical industry began developing thalidomide derivatives which are characterized be an enhanced effect with less side effects.

These IMiDs® (immunomodulatory drugs) are today used for the treatment of multiple myeloma, malignant B-cell lymphoma, leprosy and various autoimmune diseases. Nevertheless, because of the structural similarities with thalidomide these drugs are deemed to be teratogenic and cannot be prescribed for women who are pregnant or who might be conceiving. For this reason, the drug lenalidomide is only available in the United States through a restricted distribution system called RevAssist. The development of non-teratogenic thalidomide variants has so far failed because the molecular mechanism was not known and no reliable test for teratogenicity was available. EP 2 492 686 A1 discloses that a screening method for a non-teratogenic substance could be based on determining if a test substance binds to cereblon or a fragment of cereblon. Nevertheless the only concrete example of EP 2 492 686 A1 for such an assay is based on affinity purification with thalidomide-immobilized beads. This assay has the disadvantage that it based on cell extracts has long incubation time of 2 hours and for determination of the result eluate fractions have to be analyzed by SDS-PAGE and Western blotting which needs further time and complicated automatization.

There is a need for an *in vitro* test which allows reliable analyzing the teratogenic potential in humans of such newly developed pharmaceuticals but also in regard to other chemical substances people may exposed to, like herbicides, solvents or substances used in industrial processes.

It is the objective of the present invention to provide an *in vitro* assay to analyze the teratogenic potential of chemical substances, preferably pharmaceutically active substances via their binding to the protein cereblon, a cellular target of thalidomide based on direct optical determination.

The objective of the present invention is solved by the teaching of the independent claims. Further advantageous features, aspects and details of the invention are evident from the dependent claims, the description, the figures, and the examples of the present application.

The present invention provides methods for identifying binding of chemical compounds to cereblon as well as methods for preparing co-crystal forms of cereblon and their crystal structure information. The present invention relates also to the first high resolution x-ray crystal structure of a bacterial cereblon homologue together with ligands, like thalidomide or lenalidomide. The structure of the binding pocket provides data to develop methods for rational drug

design and for testing teratogenicity of the obtained active agents.

Despite extensive studies on the mechanism of action of thalidomide and its derivatives a first mechanism to the teratogenic molecular mechanism could only be characterized recently. The human Cereblon protein (CRBN) was identified as an intracellular target of thalidomide (Ito et al. Science 327:1345-1350). CRBN is part of the ubiquitin ligase complex E4 and is suggested to be a novel substrate receptor of this complex. This complex seems to play an important role in the development of limbs and the expression of BMP (bone morphogenetic protein) and FGF8 (fibroblast growth factor) in zebrafish and chicks. Teratogenic effects of thalidomide could at least partially explained by the observed inhibition of the ubiquitin ligase activity by the binding of thalidomide to CRBN in the complex thereby inhibiting the ubiquitin dependent proteolysis and thus leads to deregulation of coupled signal cascade.

In order to throw light on the molecular processes of the binding of thalidomide to cereblon the inventors have elucidated the structure of the bacterial homologue of *Magnetospirillum gryphiswaldense* in complex with thalidomide and other ligands. Based on these data, uridine was identified as a natural ligand of cereblon and binding to the protein of various therapeutically interesting substances being deemed to be teratogenic could be shown, too. These substances include basic building blocks of widespread sedatives, anticonvulsants, and anti-depressants.

Accordingly the present invention relates to a co-crystal form of cereblon homologue of *M. gryphiswaldense* having the amino acid sequence as represented by SEQ ID No. 3 with a ligand characterized as having a space group of $P2_12_12_1$, three monomers of cereblon homologue of *M. gryphiswaldense* in the asymmetric unit and unit cell dimension of $a_1$ = 55 - 58 Å, $b_1$ = 58-61 Å, $c_1$ = 85-90 Å, $\alpha_1 = \beta_1 = \gamma_1$ = 90°; or having a space group of $P3_221$, one monomer of cereblon homologue of *M. gryphiswaldense* in the asymmetric unit and unit cell dimension of $a_2 = b_2$ = 50 - 53 Å, $c_2$ = 82 - 87 *Å*, $\alpha_2 = \beta_2$ = 90° and $\gamma_2$ = 120°; or having a space group of $P6_122$, four monomers of cereblon homologue of *M. gryphiswaldense* in the asymmetric unit and unit cell dimension of $a_3 = b_3$ = 135 - 140 Å, $c_3$ = 151 - 160 Å, $\alpha_3 = \beta_3$ = 90° and $\gamma_3$ = 120°.

Said co-crystal form of cereblon homologue of *M. gryphiswaldense* is further characterized by the atomic structure coordinates of the complex with thalidomide in space group $P2_12_12_1$ in figure 10. The co-crystal forms generally comprise the cereblon homologue of *M. gryphiswaldense* with a ligand bound to the binding pocket.

The term "unit cell" as used herein refers to the smallest repeating unit that can generate a crystal with only translation operations. The unit cell is the smallest unit of volume that contains all of the structural and symmetry information of a crystal and that by translation can reproduce a pattern in all of space. Structural information is thereby the pattern (atoms) plus all surrounding space and symmetry information means mirrors, glides, axes, and inversion centers. The translation refers to motion along a cell edge the length of the cell edge. An asymmetric unit is the smallest unit that can be rotated and translated to generate one unit cell using only the symmetry operators allowed by the crystallographic symmetry. The asymmetric unit may be one molecule or one subunit of a multimeric protein, but it can also be more than one. Hence the asymmetric unit is the smallest unit of volume that contains all of the structural information and that by application of the symmetry operations can reproduce the unit cell. The shape of the unit cell is constrained by the collection of symmetry elements which make-up the group. For space groups, seven lattice types are possible: triclinic, monoclinic, orthrombic, tetragonal, hexagonal, rhombic and cubic. In crystallography, space groups are also called the crystallographic or Fedorov groups.

The edges of the unit cell define a set of unit vector **a**, **b**, and **c**, with the unit cell dimensions as their respective length a, b, and c (cf. figure 11). The angels between these vectors don't have to be right angels, and are denoted $\alpha$ (between the **bc-**vectors), $\beta$ (between the **ac**-vectors), and $\gamma$ between the (**ab**-vectors).

The term "ligand" refers to any compound, which specifically binds to the binding pocket of cereblon homologue of *M. gryphiswaldense* as shown by figure 1 and 7. It is preferred within this invention that the ligands are selected of the group comprising or consisting of thalidomide, lenalidomide, pomalidomide, deoxyuridine, hydantoin, glutarimide, ethosuximide, and aminoglutethimide. Especially preferred as a ligand is thalidomide and its derivatives and especially the derivatives of the group named IMiDs® like lenalidomide (CC-5013) and pomalidomide (CC-4047).

One aspect of the present invention is the co-crystal form of cereblon homologue of *M. gryphiswaldense* with a ligand characterized as having a space group of $P2_12_12_1$, three monomers of cereblon homologue of *M. gryphiswaldense* in the asymmetric unit and unit cell dimension of $a_1$ = 55 - 58 Å, $b_1$ = 58-61 Å, $c_1$ = 85-90 Å, $\alpha_1 = \beta_1 = \gamma_1$ = 90°.

Another aspect of the present invention is the co-crystal form of cereblon homologue of *M. gryphiswaldense* and a ligand having a space group of $P3_221$, one monomer of cereblon homologue of *M. gryphiswaldense* in the asymmetric unit and unit cell dimension of $a_2 = b_2$ = 50 - 53 Å, $c_2$ = 82 - 87 *Å* $\alpha_2 = \beta_2$ = 90° and $\gamma_2$ = 120°.

One further embodiment of the present invention refers to the co-crystal form of cereblon homologue of *M. gryphiswaldense* and a ligand having a space group of $P6_122$, four monomers of cereblon homologue of *M. gryphiswaldense* in the asymmetric unit and unit cell dimension of $a_3 = b_3$ = 135 - 140 Å, $c_3$ = 151 - 160 Å, $\alpha_3 = \beta_3$ = 90° and $\gamma_3$ = 120°.

In another embodiment of the present invention the co-crystal form of cereblon homologue of *M. gryphiswaldense* in complex with thalidomide is characterized as having a space group of $P2_12_12_1$ and three monomers of cereblon homologue of *M. gryphiswaldense* in the asymmetric unit, wherein $R_1$ represents B; has unit cell dimension of a = 56.5 Å, b = 60.1 Å, c = 88.5 Å, and $\alpha = \beta = \gamma$ = 90.0°.

One further embodiment of the present invention refers to a co-crystal of the present invention comprising at least the thalidomide binding domain of the cereblon homologue of *M. gryphiswaldense,* as defined by structure coordinates of a set of amino acid residues that correspond to amino acid residues 18-124 according to SEQ ID NO. 1 or respectively to SEQ ID NO. 8, wherein the root mean square deviation of the backbone atoms of the amino acids with respect to the structure of the cereblon homologue of *M. gryphiswaldense* reported herein is not more than 3 Å.

Another embodiment of the present invention relates to a method for co-crystallizing cereblon homologue of *M. gryphiswaldense* or a peptide comprising at least the thalidomide binding domain of the cereblon homologue of *M. gryphiswaldense* and a ligand, preferred thalidomide, comprising

i. providing a buffered, aqueous solution containing cereblon homologue of *M. gryphiswaldense;*
ii. adding a ligand, preferred thalidomide, to the aqueous solution of cereblon homologue of *M. gryphiswaldense*
iii. growing co-crystal forms by mixing the mixture of step ii) with a reservoir solution of pH 3.5 - 8.0 comprising between 5 % and 30 %(w/v) of a precipitant and equilibrate against the reservoir solution.

Thereby it is preferred that the buffered, aqueous solution of cereblon homologue of *M. gryphiswaldense* has a pH between 3.5 and 8.0 and preferably between pH 7.0 and 8.0. Furthermore the amount of the cereblon homologue of *M. gryphiswaldense* in the buffered, aqueous solution of step (i) is preferably between 0.1 mg/mL and 100 mg/L, more preferred between 1 mg/mL and 50 mg/L, and even more preferred between 2 mg/mL and 20 mg/L.

Preferred are methods for co-crystallizing cereblon homologue of *M. gryphiswaldense* and a ligand wherein the ligand is added to the aqueous solution of the cereblon homologue of *M. gryphiswaldense* to a final concentration of 0.05 mM to 20 mM of the ligand, preferably of 0.1 to 10 mM and most preferably of 1 mM. The ligand may be added in form of a 100mM stock solution for example in 100% DMSO or in any other solvent suitable for the specific ligand, or the ligand may be added as a solid substance (for example after cryo lyophilization).

The reservoir solution used in step (ii) contains between 5 % and 30 %(w/v) of a precipitant and more preferred between 10 % and 25 %(w/v). The general function of precipitants in protein crystallization experiments is to decrease the solubility of the protein. In theory, the precipitants compete with the protein solutes for water, thus leading to supersaturation of the proteins. Salts, polymers, and organic solvents are suitable precipitants. The precipitant used in the reservoir solution of step (ii) may preferably be selected from the group consisting of or comprising: 2-methyl-2,4 pentanediol, glycerol, sodium fluoride, calcium chloride, ammonium sulfate or phosphate, lithium or sodium sulfate, lithium chloride, sodium or ammonium citrate, sodium or potassium phosphate, sodium or potassium chloride, sodium or ammonium acetate, ammonium chloride, cetyltrimethyl ammonium salt, ehtylene glycol, polyethylene glycol (PEG), or mixtures thereof. Most preferred as the precipitant used in the method for co-crystallizing of the present invention is PEG, preferably having molecular weights range from PEG200 to PEG20,000. Organic solvents, like isopropanol or 1,3-propanediol may also be part of the reservoir solution.

Preferred are methods for co-crystallizing cereblon homologue of *M. gryphiswaldense* and a ligand via the hanging- or sitting-drop vapor diffusion method. Here a drop of small volume containing a mixture of the protein solution supplemented with a ligand from step (ii) and reservoir solution (also called precipitant solution) from step (iii) is equilibrated through the vapor phase against a larger volume of the reservoir solution (reservoir). The volume of the drop is preferably between 0.1 μL protein/ligand solution + 0.1 μL reservoir solution and 5 μL protein/ligand solution +5 μL reservoir solution. The reservoir contains preferably between 20 μL and 1000 μL reservoir solution. The equilibration against the reservoir is preferably done at 18 - 22°C and most preferably at 20°C. Another embodiment of the present invention relates to co-crystal forms of cereblon homologue of *M. gryphiswaldense* obtainable by method described above.

Alternatively, the co-crystal form of the invention may be obtained by techniques well-known in the art of protein crystallography, including batch, liquid bridge, free interface diffusion, dialysis, and vapor diffusion methods.

In the present invention co-crystal form of the cereblon homologue of *M. gryphiswaldense* comprises a binding pocket. The association of natural ligands or substrates with the active binding pockets of their corresponding receptors or binding partners is the basis of many biological mechanism of action. Similarly, many drugs exert their biological effects and unwanted side effects through association with the binding pockets or binding sites of proteins. An understanding of such associations can be used to design active agents having more favourable associations with their target, and thus, improved biological effects or having less or no associations with their target, and thus decreased unwanted side effects. Therefore, this information is valuable in designing potential drugs. The binding pocket of cereblon is important for drug design because binding to cereblon is assumed to be essential for the unwanted teratogenic effect of some active agents, like thalidomide. Figure 7 depicts the binding pocket of *M. gryphiswaldense* together with different ligands.

Thus, a further embodiment of the present invention refers to co-crystal form, comprising at least the thalidomide binding domain defined by structure coordinates of a set of amino acid residues that correspond to cereblon homologue of *M. gryphiswaldense* acid residues G18 - D124 according to SEQ ID NO. 1 together with a bound ligand, wherein the

root mean square deviation of the backbone atoms of the amino acids with respect to the structure of cereblon homologue of *M. gryphiswaldense* reported herein is not more than 3 Å. The term "root mean square deviation" means the square root of the arithmetic mean of the squares of the deviations from the mean. These residues are identified using the program Sybyl.

A further embodiment of the present invention refers to the use of a co-crystal form of the invention for determination of the three-dimensional structure of the cereblon homologue of *M. gryphiswaldense* in combination to a possible or known ligand. Another aspect of the present invention is the use of the crystal form to identify potential ligands to cereblon. The inventors found that the co-crystals of the cereblon homologue of *M. gryphiswaldense* and a ligand, in particular thalidomide, obtained after the method of the invention are surprisingly well suited for exchanging the original ligand with a different ligand (replacement soaking). Thereby, the ligand which has been part of the crystal is replaced by another ligand soaking into pre-grown protein crystals.

Therefore the invention refers to methods for identifying a compound that interacts with cereblon homologue of *M. gryphiswaldense* or a peptide comprising at least the binding pocket of the cereblon homologue of *M. gryphiswaldense,* comprising the step of

I) providing a co-crystal form according to claim 12 or 13.
II) providing a soaking solution of 1 - 10 mM of a test substance in a solution
III) incubating the crystals of step I) with the soaking solution of step III) for a time sufficient that individual monomers in the unit cells of the co-crystal exchange the ligand by the test substance.

Preferred are methods for identifying a compound that interacts with cereblon homologue of *M. gryphiswaldense,* wherein the test substance is dissolved in the reservoir solution used to grow the crystal. If not water soluble, the test substance can first be dissolved in a solvent like DMSO or acetonitrile and then be diluted in reservoir solution. Furthermore the incubation of the crystal in the soaking solution should last at least 6h; preferably at least 12h and even more preferred more than 24h.

Thereby the atomic coordinate/x-ray diffraction data of the co-crystal may be used to create a physical model with and without the ligand which can then be used to design molecular models of compounds or derivatives of known active agents having teratogenic effects that may not longer interact with the determined binding pocket or other structural or functional domains or subdomains of cereblon. Alternatively, the atomic coordinate/x-ray diffraction data of the cereblon homologue or the binding pocket thereof may be represented as atomic model output data on computer readable media which can be used in a computer modeling system to calculate different molecules expected to interact with the determined binding sites, or other structural or functional domains or subdomains of cereblon, preferably the thalidomide binding pocket. For example, computer analysis of the data allows calculating the three-dimensional interaction of cereblon and a ligand to confirm that the ligand binds to cereblon or does not bind, which is preferred. Compounds being identified from the analysis of the physical or computer model to be no ligand can then be synthesized and tested for binding activity within the assay of the present application.

A variety of commercially available software programs are suitable for conducting the analysis and comparison of data in the computer-based system. One skilled in the art will readily recognize which of the available algorithms or implementing software packages for conducting computer analyses can be utilized or adapted for use in the computer-based system. Suitable software that can be used to view, analyze, design, and/or model a protein comprise MOLOC (Roche,1985), FRED, MAIN, FlexX, Gold, XtalView, Alchemy TM, LabVision TM, Sybyl TM, Molcadd TM, Leapfrog TM, Matchmaker TM , Genefold TM and Sitel TM (available from Tripos Inc., St. Louis, MO.); Quanta TM, Cerius2 TM, X-Plor TM, CNS TM, Catalyst TM , Modeller TM, ChemX TM, Ludi TM, Insight TM, Discover TM, Cameleon TM and Iditis TM (available from Accelrys Inc., Princeton NJ); Rasmol TM (available from Glaxo Research and Development, Greenford, Middlesex, U.K.); MOE TM (available from Chemical Computing Group, Montreal, Quebec, Canada); Maestro TM (available from Shrdinger Inc.,); Midas/MidasPlus TM (available from UCSF, San Francisco, CA); JyMol, Pymol (Schrödinger LLC); Jmol (freeware on the intemet); VRML, (webviewer- freeware on the internet); Chime (MDL - freeware on the intemet); AutoDock (available from The Scripps Research Institute); MOIL (available from University of Illinois, Urbana-Champaign, IL); MacroModel TM and GRASP TM (available from Columbia University, New York, NY); Ribbon TM (available from University of Alabama, Tuscaloosa, Alabama); NAOMI TM (available from Oxford University, Oxford, UK); Explorer Eyechem TM (available from Silicon Graphics Inc., Mountain View, CA.); Univision TM (available from Cray Research Inc., Seattle WA); Molscript TM and O (available from Uppsala University, Uppsala, Sweden); Chem 3D TM and Protein Expert TM (available from Cambridge Scientific); and upgraded versions thereof.

Further embodiment of the present invention is a method for identifying a compound that does not interact with cereblon, comprising the step of

(1) generating a three-dimensional model of the cereblon homologue of *M. gryphiswaldense* using the structure coordinates as listed in figure 10, and

(2) employing said three-dimensional model to design or select a compound that does not interact with cereblon.

It is preferred, that this method is based on a computer-assisted method.

Based on the solved co-crystal structures of the cereblon homologue of *M. gryphiswaldense* the inventors could prove that three tryptophan residues of the binding pocket of cereblon may be used for an assay detecting binding to cereblon. Based on these data the inventors developed a high throughput assay which allows the in vitro analysis of potential teratogenic effects based on the binding to cereblon (CRBN). This method represents a quick, cost-effective assay for routine controls of teratogenic potential of candidate substances.

Thus one aspect of the invention refers to a method of identifying substances which bind to a thalidomide binding peptide based on determining alteration of tryptophan fluorescence within the binding pocket of the thalidomide binding peptide. Said inventive methods refer to assays completely practicable *in vitro.* Because of the elucidation of the molecular and structural basis for the binding of thalidomide to cereblon using crystallography the inventors could show that a variety of pharmacologically relevant compounds that are already classified as teratogens are bound in identical manner as thalidomide and uracil (Figure 7), which is also reported to be teratogenic (cf. 9th edition of Sax's Dangerous Properties of Industrial Materials).

In general it is possible to test for a possible quenching or dequenching (increasing) effect of the candidate substance on the tryptophan fluorescence of the thalidomide binding peptide. The term "quenching" as used herein refers to any process which decreases the fluorescence intensity of the used thalidomide binding peptide. A variety of processes can result in quenching, such as excited state reactions, energy transfer, complex-formation and collisional quenching. Furthermore the emission wave length of the tryptophan residues of the thalidomide binding peptide may be changed due to the binding of the test compound. One aspect of the invention refers to a method of identifying substances which bind to a thalidomide binding peptide comprising the following steps:

A) providing a thalidomide binding peptide
B) providing a candidate substance to be tested
C) forming a reaction mixture by contacting the candidate substance with the thalidomide binding peptide
D) incubating the reaction mixture under conditions sufficient to allow the candidate substance to bind to the thalidomide binding peptide, in case the candidate substance is able to bind to the thalidomide binding peptide,
E) determining if the candidate substance forms a complex with the thalidomide binding peptide using alteration in tryptophan fluorescence within the binding pocket of the thalidomide binding peptide.

The term "thalidomide binding peptide" as used herein refers to a polypeptide thalidomide specifically binds to and is described in greater detail below. The candidate substance to be tested within a method of the invention for binding to a thalidomide binding polypeptide and therefore to the human cereblon may be each chemical molecule. Determination of the teratogenic potential of substances is of general interest for all substances a human may be exposed to.

Additionally, it is possible to add not only the test substance but also another component which should be analyzed in regard to its influence on the binding of the test substance to the thalidomide binding peptide. Nevertheless preferred are methods according to the invention, wherein the candidate substance is an imide or a lactam, more preferred a 5- or 6-membered cyclic imide or a γ- or δ-lactam. More preferred are substances, like pharmaceutically active agents, derived from thalidomide, glutarimide, hydantoin, succinimide, oxazolidine-2,4-dione, 2-pyrrolidone and uracil. Especially preferred are substances derived from thalidomide. Substances of these classes are for example know as immunomodulatory substances, anticonvulsants or antiepileptic drugs, but also as disinfectants, plasticizer or component of industrial production processes, like silver coating processes.

Step C) of the inventive method refers to formation of a reaction mixture by contacting the candidate substance with the thalidomide binding peptide. Step C) of the inventive method could also be defined as mixing the thalidomide binding peptide of step A) with the candidate substance. Step C) as well as the complete method of the invention could be performed in solution. This means that the thalidomide binding peptide of step A) and the candidate substance may be solved together in one solution or that a solution of the thalidomide binding peptide is mixed with a solution of candidate substance. Such a solution could be based on any suitable solvent as well as buffer solutions or a mixture of a solvent, especially an organic solvent and a buffer. It should be avoided that the solvent has a quenching effect interfering with the inventive method and that the solvent or buffer denatures the thalidomide binding peptide. Hence denaturants, surfactants or other amphiphilic molecules in the buffer should be avoided.

Preferably the candidate substance in solid form or a solution of the candidate substance is added. Thereby it is preferred that different concentrations of the candidate substance will be added in a way that a concentration series is formed. The evaluation of a concentration series may allow calculating the affinity of the candidate substance for binding to cereblon. Depending on the binding affinity it may be useful to determine an optimal concentration range for the assay in advance. One embodiment of the present invention refers to a method comprising step C) wherein different

concentrations of the candidate substance are added to the thalidomide binding peptide (each concentration to one sample containing thalidomide binding peptide) which allows a quantitative determination of the binding of the candidate substance after data analysis. The aim is to find candidate substance having an affinity to cereblon which is remarkably lower than the affinity of thalidomide. The ideal substance would be a compound having at least one biological or pharmaceutical effect of thalidomide but showing no binding to cereblon or, respectively, the thalidomide binding peptide.

Step D) refers to incubation of the reaction mixture under conditions sufficient to allow the candidate substance to bind to the thalidomide binding peptide, in case the candidate substance is able to bind to the thalidomide binding peptide. Forming a complex occurs by intermolecular forces, such as ionic bonds, hydrogen bonds and van der Waals forces. The docking is preferably reversible. During incubating a complex of the thalidomide binding peptide of step A) with the candidate substance takes place spontaneously, in case the candidate substance is able to bind to the thalidomide binding peptide. Preferably step D) comprises incubation at room temperature for some minutes, approximately 10 minutes.

A solvent for the candidate substance should not quench or interfere with a component of the inventive method. Denaturation of the peptide should be avoided; therefore temperature should not be higher than 50°C. Furthermore there may be included a solvent or mixture of different solvents, a buffer system or optionally further additives like protease inhibitors.

Step E) refers to determination if the candidate substance forms a complex with the thalidomide binding peptide using alteration in tryptophan fluorescence within the binding pocket of the thalidomide binding peptide. Determining if the candidate substance binds to the thalidomide binding peptide may be done by measurement of tryptophan fluorescence wherein occurrence of a quench effect indicates binding of the candidate substance to the thalidomide binding peptide. Fluorescence may be analyzed using common fluorescence spectroscopy or spectrofluorometry, a type of electromagnetic spectroscopy. The preferred wavelength for the measurement is therefore around 295 nm excitation and 350 - 370 nm emission.

One further aspect of the invention refers to a method of identifying substances which bind to a thalidomide binding peptide comprising the following steps:

A) providing a thalidomide binding peptide
B) providing a candidate substance to be tested
C) forming a reaction mixture by contacting the candidate substance with the thalidomide binding peptide
D) incubating the reaction mixture under conditions sufficient to allow the candidate substance to bind the thalidomide binding peptide, in case the candidate substance is able to bind to the thalidomide binding peptide,
E) determining if the candidate substance binds to the thalidomide binding peptide by measurement of fluorescence wherein a quench effect indicates binding to a thalidomide binding peptide.

It could be shown, that tryptophan fluorescence is a very sensitive measurement of the state of binding to cereblon. The advantage of determining the intrinsic tryptophan fluorescence compared to extrinsic probes is that the protein itself is not changed (e.g. tag). A change in the intensity of the tryptophan fluorescence indicates binding of the candidate substance to the thalidomide binding peptide. Binding to the thalidomide binding peptide, respectively cereblon, correlates to the teratogenic potential of the candidate substance. In case a concentration series is measured $K_d$ or $IC_{50}$ and $K_i$ values for candidate substances can be determined if the measured values of fluorescence follow a binding curve (or dose-response-curve) being a saturation curve. This means that the binding reaches a maximum within titration with increasing amounts. Furthermore it could be that a change in fluorescence intensity or wavelength occurs but that a linear binding curve is the result of the concentration series. Reason for a nearly linear binding curve could be unspecific binding of the candidate substance or that another compound of the reaction mixture interacts with the fluorescence. No alteration in fluorescence intensity indicates that no binding occurs or that the candidate does not interact with the tryptophan fluorescence of the thalidomide binding peptide. Therefore it is preferred that the method comprises further verification steps at least if no saturation binding curve can be observed. One can for example use the principle of competitive binding assays by using a specific binding agent which competes for the binding pocket of the thalidomide binding peptide.

Hence, optionally, the method described above comprises further the following steps F) - H):

F) adding a compound binding to said thalidomide binding peptide and having an excitation frequency between 310 and 425 nm.
G) incubating the reaction mixture under conditions sufficient to allow the compound of step F) to replace the candidate substance in the complex, and
H) determining if the candidate substance has been replaced in the complex compound of step F).

The compound of step F) is the specific binding agent which competes for the binding, more specific binding

to the thalidomide binding pocket of the thalidomide binding peptide. Suitable compounds are characterized by two features: they should be a fluorescent compound being able to be excited by the fluorescence of the tryptophan residues of the binding pocket of the thalidomide binding peptide and being a known ligand of the thalidomide binding peptide. The compound of step F) corresponds to the compound of step b) as described below. Therefore all statement concerning compound of step b) being a compound binding to said thalidomide binding peptide and having an excitation frequency between 310 and 425 nm applies to the compound of step F), too. If the candidate substance has formed a complex with the thalidomide binding peptide (during step F)) at least in some complexes the compound of step F) replaces the candidate substance. The incubation means that the reaction mixture is again incubated for some time, preferably between 2 and 30 minutes, more preferred between 10 and 20 minutes. The term "suitable conditions" refers mostly to a suitable temperature, preferably at room temperature, and a suitable solvent or buffer used in the reaction mixture which is more or less the same then before. The compound of step F) may be added as a solid substance or being in solution. It is preferred that the compound of step F) is solved in the solvent or buffer of the reaction mixture or that the added solution contains the compound of step F) very concentrated so that only less volume is added. In general the solvent of step F) can be each solvent suitable for solving compound of step F) and which does not (at least not in the added concentration) interfere with fluorescence.

Step H) of the inventive methods comprises determining if the candidate substance has been replaced in the complex compound of step F). This may be done using photometry. The specific arrangement of three tryptophan residues in the binding pocket of the thalidomide binding peptide, allows using the FRET effect to determine and characterize binding to cereblon. Because the principle of FRET needs spatial proximity binding of the compound of step F) which is excitable at the emission wavelength of the tryptophan fluorescence replacement of the candidate substance is indicated by FRET taking place. Step H) of the preferred embodiment of the inventive method refers therefore to determining fluorescence at a specific wave length, namely the emission maximum of the compound of step F). An increase in fluorescence indicates a replacement of the candidate substance which is indicative for a teratogenic potential of the candidate substance.

If the emission of the compound of step F) obtained from the reaction mixture with several concentrations of the candidate substance and plotted against the candidate substance concentration shows the characteristics of a competitive binding curve but during step E) no saturation curve was detected, one can follow that the candidate substance binds but does not interact with the intrinsic fluorescence of the thalidomide binding pocket. The FRET data obtained can then be used for determination of the affinity. If also the binding curve obtained within step E) was a saturation binding curve, the additional steps could verify that the candidate substance binds specific to the binding pocket of the thalidomide binding peptide. In case the fluorescence emitted from the compound of step F) (FRET effect) has the same intensity independent of the concentration of the candidate substance the candidate substance does not bind to the thalidomide binding peptide and has no teratogenic potential based on binding to cereblon.

Nevertheless it is also possible to use directly a competitive method as described in the following.

Another aspect of the invention refers to a method of identifying substances which bind to a thalidomide binding peptide comprising the following steps:

a) providing a thalidomide binding peptide
b) providing a compound binding to said thalidomide binding peptide interacting with the tryptophan fluorescence of the thalidomide binding peptide
c) providing a candidate substance to be tested
d) forming a complex of the thalidomide binding peptide with the compound of step b)
e) forming a reaction mixture by contacting the candidate substance with the complex of step d)
f) incubating the reaction mixture under conditions sufficient to allow the candidate substance to replace the compound of step b) in the complex of step d), in case the candidate substance is able to bind to the thalidomide binding peptide,
g) determining if the compound of step b) has been replaced in the complex of step d) by the candidate substance.

Within this method replacement of the compound of step b) in the complex of step d) by the candidate substance is indicative for specific binding of the candidate compound to cereblon in the same binding pocket than thalidomide and therefore of a teratogenic potential of the candidate substance.

Three components are required for said method according to the invention: firstly a thalidomide binding peptide, i.e. the mutant W36/59F of the cereblon homologue of *M. gryphiswaldense* and secondly a compound binding to said thalidomide binding peptide, more specific to the thalidomide binding pocket of said peptide, and interacting with the tryptophan fluorescence of the thalidomide binding peptide, like 1-(N-2-MANT-imidoethyl)-uracil and thirdly, a candidate substance to be tested. Said components and their function are described in greater detail below. Thereby preferably all three components are added being in solution or at least one component is in solution and remaining components are added to this solution. This means the method or the inventive assay takes preferably place in solution. The term

"in solution" as used herein means that the thalidomide binding peptide, the compound of step b) and the test substance are preferably not bound to any carrier, like micro titer plates or beads. Therefore the three components of the inventive methods are preferably free and unbound except of the interactions among themselves.

Interacting with the tryptophan fluorescence of the thalidomide binding peptide within the present invention refers to compounds which change the fluorescence of the thalidomide binding peptide caused by three tryptophans in the binding pocket upon specific binding to that binding pocket. Interacting with the tryptophan fluorescence of the thalidomide binding peptide within the present invention comprise preferably quenching or dequenching (increasing) the fluorescence of the tryptophan residues of the thalidomide binding peptide.

Förster resonance energy transfer (FRET or FET) is a dynamic quenching mechanism because energy transfer occurs while the donor is in the excited state. One preferred aspect of the present invention is a method of identifying substances which bind to a thalidomide binding peptide based on FRET.

Accordingly, the present invention relates preferably to a method of identifying substances which bind to a thalidomide binding peptide comprising or consisting of the following steps:

a) providing a thalidomide binding peptide
b) providing a compound having an excitation frequency between 310 and 425 nm and binding to said thalidomide binding peptide
c) providing a candidate substance to be tested
d) forming a complex of the thalidomide binding peptide with the compound of step b)
e) forming a reaction mixture by contacting the candidate substance with the complex of step d)
f) incubating the reaction mixture under conditions sufficient to allow the candidate substance to replace the compound of step b) in the complex of step d), in case the candidate substance is able to bind to the thalidomide binding peptide,
g) determining if the compound of step b) has been replaced in the complex of step d) by the candidate substance.

Three components are required for said method: firstly a thalidomide binding peptide, i.e. the mutant W36/59F of the cereblon homologue of *M. gryphiswaldense* and secondly a compound having an excitation frequency between 310 and 425 nm and binding to said thalidomide binding peptide, like 1-(N-2-MANT-imidoethyl)-uracil and thirdly, a candidate substance to be tested. Said components and their function are described in greater detail below.

Cereblon is a protein that in humans is encoded by the *CRBN* gene. The human CRBN protein consists of 442 amino acids and has a molecular weight of around 50.5 kDa. The protein has on the N-terminus a natively unstructured region followed by a region with homology to the PUA as well as to the LON protease domain. This domain is followed by a region decisively for the interaction with DDB1 (DNA damage-binding protein 1). The subsequent C-terminal domain (104 C-terminal amino acids) is essential for the binding of thalidomide. Proteins with homology to said thalidomide binding domain of the human cereblon are widely spread in eukaryotes.

In contrast bacterial homologues are so far found only in three classes, namely 6-proteobacteria, γ-proteobacteria and within the genus of the α-proteobacteria. Generally, there are two groups of cereblon homologues; first group contains only eukaryotic proteins with a domain organization identical to the human cereblon. The second group comprises one-domain proteins homologue to the thalidomide binding domain. In bacteria only proteins of this second group are found. Eukaryotes until the evolutionary stage of zebrafish have mostly both forms. Higher eukaryotes like humans and *Arabidopsis* have only a multidomain cereblon protein.

The term "thalidomide binding peptide" as used herein refers to a polypeptide thalidomide specifically binds to. Peptides are compounds composed of amino acids joined together by peptide bonds between adjacent amino acids into linear, branched or cyclical structures. The thalidomide binding peptides of the present invention should be composed of at least 80 amino acids, preferred at least 90 amino acids and even more preferred at least 100 amino acids.

The "thalidomide binding peptides" as used herein have an amino acid sequence that is specifically bound by thalidomide. The binding occurs by intermolecular forces, such as ionic bonds, hydrogen bonds and van der Waals forces. The docking is usually reversible. A specific binding is characterized by a binding curve which correlates to a saturation curve. This means that the binding reaches a maximum within titration with increasing amounts. In contrast unspecific binding is characterized by a nearly linear binding curve. Binding to a protein or polypeptide may be characterized in terms of the concentration of the ligand, here thalidomide, at which half of the polypeptide binding sites are occupied, known as the dissociation constant ($K_d$). It is preferred if the thalidomide binding peptides of the present invention have a $K_d$ up to 100 μM, preferred up to 50 μM and even more preferred up to 10 μM for thalidomide. It seems that only the correctly folded thalidomide binding domain of cereblon is able to bind its ligands, like thalidomide and uridine. The crystal structure suggests that the whole thalidomide binding domain, including the regions N- and C-terminal to the binding pocket is necessary for proper folding. Preferred thalidomide binding peptides are: cereblon, a cereblon homologue, the thalidomide-binding domain of cereblon, a peptide homologous to the thalidomide binding domain of the human cereblon comprising the amino acid sequence of SEQ ID No. 7 representing the amino acids C328-L442 of

the human cereblon, or a peptide homologous to the thalidomide binding domain of the cereblon homologue of *M. gryphiswaldense* comprising the amino acid sequence of SEQ ID No. 8. The definition of homology as used herein is described below.

The term "cereblon" as used herein refers to a protein that in humans is encoded by the CRBN gene. The gene that encodes the cereblon protein is found on the human chromosome 3, on the short arm at position p26.3 from base pair 3,190,676 to base pair 3,221,394. The term "cereblon" as used herein refers also to all cereblon orthologs in eukaryotes which are highly conserved from plants to humans, retain the same function in the course of evolution and have a domain organization identical or very similar to the human cereblon.

Cereblon according to the present application refers preferably to cereblon of Arabidopsis thaliana GenBank Accession: AEC07747.1, Cereblon of Homo sapiens GenBank Accession:AAH67811.1, Cereblon of Mus musculus GenBank Accession: NP_067424.2 or NP_780566.1, Oryctolagus cuniculus (XP_002720669), Sarcophilus harrisii (XP_003762431), Strongylocentrotus purpuratus (XP_001191267), Saccoglossus kowalevskii (XP_002730537), Branchiostoma floridae (EEN53302.1), Nematostella vectensis (EDO40533.1), Ciona intestinalis (XP_002127932), Ixodes scapularis (EEC11079.1), Trichoplax adhaerens (EDV26348.1), Selaginella moellendorffii (EFJ07516.1), Daphnia pulex (EFX70803.1), Populus trichocarpa (EEE92116.1), Physcomitrella patens subsp. Patens (EDQ68142.1), Zea mays (ACL53993.1), Ricinus communis (EEF45868.1), Hordeum vulgare subsp. Vulgare (BAK00783.1), Glycine max (XP_003523752), Capsaspora owczarzaki (ATCC 30864 EFW44731.1), Tribolium castaneum (EFA08465.1), Pediculus humanus corporis (EEB15631.1), Danaus plexippus (EHJ76333.1), Physcomitrella patens subsp. Patens (EDQ59356.1), Glycine max (XP_003527849), Metaseiulus occidentalis (XP_003747722), Nasonia vitripennis (XP_001603993) Brachypodium distachyon (XP_003566024), Brugia malayi (EDP34015.1), Clonorchis sinensis (GAA33019.2), Aedes aegypti (EAT43511.1), Schistosoma mansoni (CCD75398.1), Ascaris suum (ADY42257.1), Drosophila pseudoobscura pseudoobscura (EAL27126.2), Drosophila simulans (EDX13533.1), and Coccomyxa subellipsoidea C-169 (EIE19963.1).

The term "thalidomide-binding domain of cereblon" as used herein describes a polypeptide comprising the C-terminal domain of cereblon being essential for the binding of thalidomide. This means in the inventive method not a complete cereblon is used but only the domain of the protein which is bound by thalidomide. This domain refers mainly to the amino acid residues C328-L442 of human cereblon.

The term "cereblon homologue" as used herein refers to one-domain proteins which are homologues to the thalidomide binding domain of cereblon. These proteins are found in bacteria and in eukaryotes until the evolutionary stage of zebrafish. Preferred are cereblon homologues which have no other tryptophan residues then the tryptophan residues in the binding pocket or which have at least no tryptophan residues at their surface. Alternatively, tryptophan residues disturbing measurement of the fluorescence of the tryptophan residues in the binding pockets and/or a FRET effect based on the tryptophan residues in the binding pockets may be eliminated by site directed mutagenesis.

"Sequence identity" as used herein indicates the percentage match of sequences by using alignment between two sequences. An alignment is simply a correspondence between the sequences, in which each symbol in a sequence is assigned no more than one (maybe none) of the symbols in the other sequence, and in which the order of the symbols in the sequence is maintained. Hence sequence identity is the number (%) of matches (identical characters) in positions from an alignment of two molecular sequences. In this application, two sequences are called "homologous" if their sequence alignment has an Expect value (E-value) better than 1.0e-5 as defined by the blast algorithm as implemented at http://blast.ncbi.nlm.nih.gov (blast: *Basic Local Alignment Search Tool*). Therefore, a peptide is herein said to be homologous to the thalidomide binding domain of the human cereblon if its alignment with the thalidomide binding domain of the human cereblon yields an E-value better than 1.0e-5.

It is preferred if the cereblon homologue is a bacterial cereblon homologue, preferably the cereblon homologue is selected from the group comprising or consisting of Desulfovibrio fructosovorans JJ (ZP_07333566.1), Desulfovibrio aespoeensis Aspo-2(YP_004119828.1), Desulfovibrio salexigens DSM 2638 (YP_002992124.1), Desulfovibrio desulfuricans ND132 (YP_005168109.1), Desulfovibrio africanus str. Walvis Bay|YP_005053025.1), Desulfococcus oleovorans Hxd3 (YP_001528511.1), Desulfohalobium retbaense DSM 5692|YP_003197581.1), Desulfobacula toluolica Tol2 (YP_006761206.1), Desulfobacter postgatei 2ac9 (ZP_10171192.1), Desulfobacterium autotrophicum HRM2 (YP_002603710.1), Desulfomicrobium baculatum DSM 4028 (YP_003159665.1), Haliangium ochraceum DSM 14365|YP_003270892.1), uncultured bacterium (EKD36519.1), Beggiatoa alba B18LD|ref|ZP_101 13358.1) and gamma proteobacterium BDW918 (ZP_10064520.1). A preferred method according to the invention uses a bacterial homologue of cereblon as the thalidomide binding peptide and a method even more preferred uses a W36/59F mutant of *M. gryphiswaldense* of SEQ ID No. 6 as thalidomide binding peptide. This indication means that in this mutant a tryptophan is replaced by a phenylalanine at position 36 and also at position 59, counting from the N-terminal end of the wild type protein sequence represented by SEQ ID No. 1.

Another preferred thalidomide binding peptide in regard to the inventive method is the cereblon-homologue of *C. elegans.* Especially preferred is the thalidomide binding peptide represented by SEQ ID No. 9. The thalidomide binding peptide having sequence of SEQ ID No. 9 differs from the wild type protein by lacking the first 15 amino acid residues at the N-terminus and having one additional methionine followed by an additional glycine at the N-terminus and a TEV-protease cleaving site at the C-terminus as a remainder from the cleaved His-tag.

The term "cereblon polypeptide" refers to the protein cereblon (CRBN) or its eukaryotic or bacterial homologue that minimally comprises the thalidomide binding domain of cereblon, while the term "cereblon nucleic acid" refers to a nucleic acid such as DNA or RNA, mRNA encoding the protein cereblon or a cereblon polypeptide.

One aspect of the invention is a mutant cereblon homologue of *M. gryphiswaldense* with an amino acid sequence having at least a modification of amino acid residues 36 and 59 of SEQ ID No. 1. It is especially preferred if the amino acid residues 36 and 59 of SEQ ID No. 1 are replaced by phenylalanine. Therefore one aspect of the invention refers to the mutant cereblon homologue of *M. gryphiswaldense* represented by SEQ ID No. 6. Said modification may be introduced by common known techniques of site-specific mutagenesis like PCR site-directed mutagenesis or Quik-Change® Site-Directed Mutagenesis. They can also be introduced by artificial synthesis of the mutated gene (gene synthesis).

One further aspect of the invention refers to said mutant cereblon homologue of *M. gryphiswaldense* being encoded by a polynucleotide comprising a nucleic acid sequence of SEQ ID No. 5, wherein the nucleotide sequence has a genetic modification of at least codon 36 and codon 59 whereas codon numbers correspond to the wild type nucleotide sequence SEQ ID No. 2. Another aspect of the invention is the cereblon nucleic acid molecules of sequence of SEQ ID No. 2, wherein at least codon 36 and codon 59 have a genetic modification or an expression vector containing said nucleic acid molecule. It is especially preferred if the modified codon 36 and 59 of SEQ ID No. 2 encodes a phenylalanine. Therefore one aspect of the invention refers to the mutant cereblon homologue of *M. gryphiswaldense* represented by the sequence of SEQ ID No. 6 and being encoded by a polynucleotide comprising a nucleic acid sequence of SEQ ID No. 5 and to the mutated cereblon nucleic acid molecules represented by the sequence of SEQ ID No. 5.

The gene of the cereblon homologue of *M. gryphiswaldense* has been codon optimized and produced by gene synthesis for expression in *Escherichia coli* producing high yields. Due to cloning requirements an additional glycine has been introduced in position 2. Therefore, one further aspect of the invention refers to a nucleic acid molecule comprising a sequence as set forth in SEQ ID No. 4 that encodes a cereblon homologue of *M. gryphiswaldense* or a nucleic acid having at least 35%, preferably at least 50%, more preferably at least 60%, more preferably at least 75%, even more preferably at least 80% and most preferably at least 90% sequence identity to SEQ ID No. 4, the nucleic acid sequence being codon-optimized for high level expression in *E. coli.*

For the expression of the corresponding polypeptides the nucleic acid sequences can be inserted into expression vectors, such as recombinant bacteriophage, plasmid, or cosmid DNA expression vectors. Therefore the present invention refers also to nucleic acid molecules containing one of the above mentioned nucleic acids, such as expression vectors. This expression vectors could also encode for fusion polypeptides of the respective cereblon polypeptide and a tag suitable for isolation of the polypeptide, like ($His_6$)-Tag. Such tag could be cleavable using a protease cleaving site between the cereblon polypeptide and the tag.

The term "peptide having at least 80% sequence identity to the thalidomide binding domain of the human cereblon" as used herein refers to peptides having a sequence which shares at least 80% of their amino acids with the sequence represented by SEQ ID No. 8. The same applies analogous to the other percentages mentioned. The same definition applies to DNA and especially codon-optimized DNA.

Thereby it is preferred that a peptide homologous to the thalidomide binding domain of the human cereblon has tryptophan amino acid residues corresponding to position 63, 69, 83 and tyrosine or phenylalanine corresponding to position 85 in the sequence of SEQ ID No. 7 and a peptide homologous to the cereblon homologue of *M. gryphiswaldense* has tryptophan amino acid residues corresponding to position 62, 68, 82 and tyrosine or phenylalanine corresponding to position 84 in the sequence of SEQ ID No. 8.

Therefore the present invention refers also to methods, wherein the thalidomide binding peptide is chosen from cereblon, a cereblon homologue, the thalidomide-binding domain of cereblon, a peptide homologous to the thalidomide binding domain of cereblon having the amino acid sequence of SEQ ID No. 7, or a peptide homologous to the thalidomide binding domain of the cereblon homologue of *M. gryphiswaldense* comprising the amino acid sequence of SEQ ID No. 8.

Step b) of the inventive method relates to compounds binding to said thalidomide binding peptide and interacting with the tryptophan fluorescence of the thalidomide binding peptide. Suitable compounds are characterized by two features: they should specifically bind to the binding pocket of thalidomide binding peptide and they should influence or interact with the intensity or wavelength of the fluorescence of tryptophan residues in said binding pocket.

One possible mechanism is static quenching (also referred to as contact quenching). Static quenching occurs when the compound of step b) binds to the thalidomide binding peptide, forming a complex with the tryptophan residues that has fluorescent properties deviating from the uncomplexed form. Planar aromatic substances can enhance static quenching. Such a quenching compound can also be a dark quencher, which absorbs excitation energy from a fluorophore, here the tryptophan residues of the binding pocket, and dissipates the energy given off via molecular vibrations (heat). This heat is thereby too small to affect the temperature of the solution. Possible quencher is dabcyl which quenches effectively over a broad wavelength range from about 360-560 nm. Therefore one possible compound of step b) is a dabcyl conjugated to a ligand of cereblon (e.g. uracil).

Step b) of the inventive method relates preferably to compounds having an excitation between 310 and 425 nm and binding to a thalidomide binding peptide, more specific binding to the thalidomide binding pocket of the thalidomide binding peptide. Suitable compounds are characterized by two features: they should be a fluorescent compound being able to be excited by the fluorescence of the tryptophan residues of the binding pocket of the thalidomide binding peptide and being a ligand of the thalidomide binding peptide. Said compound could therefore preferably comprise or consist of at least two subunits. Step b) of the inventive method relates to a compound of at least two subunits or parts, one being a fluorophore having an excitation between 310 and 425 nm and the second subunit binding to or interacting with said thalidomide binding peptide. Furthermore these subunits or parts may be conjugated via a linking moiety.

For observation and analysis of a binding or interaction between cereblon and a candidate substance or potential ligand the inventive method uses preferably the principle of Förster (Fluorescence) resonance energy transfer (FRET). This is a mechanism of energy transfer between two chromophores. A donor chromophore, initially in its electronic excited state, may transfer energy to an acceptor chromophore. The efficiency of this energy transfer is inversely proportional to the sixth power of the distance between donor and acceptor making FRET extremely sensitive to small distances. Therefore measurements of FRET efficiency can be used to determine if two chromophores or here two fluorophores are within a certain distance of each other (0.5 to 10 nm).

In the method or assay according to the present invention FRET could take place between the fluorescence of the excited tryptophan residues in the binding pocket (excitation: 295 nm) of the thalidomide binding peptide and a fluorophore or fluorescent part of the compound of step b) (for MANT-uracil excitation: 350 nm, emission: 440 nm).

The fluorescence emission of a free tryptophan has a wavelength of 350 nm. The emission spectrum shifts to shorter wavelength and the intensity of the fluorescence increases as the polarity of the solvent surrounding the tryptophan residue decreases. Tryptophan residues which are buried in the hydrophobic core of proteins can have spectra which are shifted by 10 to 20 nm compared to tryptophans on the surface of the protein. Furthermore tryptophan fluorescence can be quenched by neighboring protonated acidic groups such as Asp or Glu. The tryptophan residues in the binding pocket of a thalidomide binding polypeptide, like cereblon, have an emission between 310 and 425 nm with a maximum at 375 nm.

Therefore the compounds of step b) respectively the fluorophore or the fluorescence part of these compounds of the inventive method may be derivatives of a fluorescence molecule having an excitation frequency between 310 and 425 nm, preferably between 320 and 415 nm, more preferred between 330 and 395 nm and mostly preferred between 350 and 380 nm. This molecule may be selected from the group comprising or consisting of all fluorophores listed in table 1. It is preferred if the fluorescence molecule or fluorophore of the compound of step b) is MANT, ATTO 390 (ATTO-TEC GmbH), EDANS (5-((2-Aminoethyl)amino)naphthalene-1-sulfonic acid), Alexa Fluor® 350 (Life Technologies ), AMCA (aminomethylcoumarin acetate) or 5-HAT (Hydroxy Tryptamine).

Table 1:

| Fluorophore | excitation | emission |
|---|---|---|
| 1,5 IAEDANS | 336 | 490 |
| 1,8-ANS | 372 | 480 |
| 4-Methylumbelliferone | 385 | 502 |
| 5-Hydroxy Tryptamine (HAT) | 370-415 | 520-540 |
| 7-Amino-4-methylcoumarin | 351 | 430 |
| 7-Hydroxy-4-methylcoumarin | 360 | 49,455 |
| 9-Amino-6-chloro-2-methoxyacridine | 412,430 | 471,474 |
| ABQ | 344 | 445 |
| ACMA (9-Amino-6-chloro-2-methoxyacridine) | 412,430 | 471,474 |
| Acriflavin Feulgen SITSA | 355-425 | 460 |
| Alexa Fluor 350™ | 346,342 | 442 441 |
| AMC, AMCA-S | 345 | 445 |
| AMCA (Aminomethylcoumarin) | 345 347 | 425 444 |
| AMCA-X | 353 | 442 |
| Aminocoumarin | 346 350 | 442 445 |

(continued)

| Fluorophore | excitation | emission |
|---|---|---|
| Aminomethylcoumarin (AMCA) | 345 347 | 425 444 |
| Anthrocyl stearate | 360-381 | 446 |
| APTS | 424 | |
| BAO 9 (Bisaminophenyloxadiazole) | 365 | 395 |
| Beta Lactamase | 409 | 447,520 |
| BFP blue shifted GFP (Y66H) and Blue Fluorescent Protein | 381,382,383 | 445,447, |
| Bimane | 398 | 490 |
| Bisbenzamide | 360 | 461 |
| Bisbenzimide (Hoechst) | 360 | 461 |
| bis-BTC = Ratio Dye, Zn2+ | 455/405 | 529/505 |
| Blancophor FFG | 390 | 470 |
| Blancophor SV | 370 | 435 |
| Calcein Blue | 373 | 440 |
| Calcofluor White | 385,395,405 | 437,440,445 |
| Cascade Blue™ | 377 398 399 | 420 423 |
| Cascade Yellow | 399 400 | 550 552 |
| Catecholamine | 410 | 470 |
| Coumarin Phalloidin | 387 | 470 |
| CPM Methylcoumarin | 384 | 469 |
| CTC | 400-450 | 602 |
| Dansyl | 340 | 578 |
| Dansyl Amine | 337 | 517 |
| Dansyl Cadaverine | 335 | 518 |
| Dansyl Chloride | 372 | 518 |
| Dansyl DHPE | 336 | 517 |
| Dansyl fluoride | 356 | none |
| DAPI | 359 | 461 |
| Dapoxyl | 403 | 580 |
| Dapoxyl 2 | 374 | 574 |
| Dapoxyl 3 | 373 | 574 |
| DIDS | 341 | 415 |
| Dinitrophenol | 349 | |
| DNP | 349 | |
| Dopamine | 340 | 490-520 |
| EBFP (Enhanced Blue Fluorescent Protein) | 383 | 447 |
| ELF 97 | 345 | 530 |
| Fast Blue | 360 | 440 |
| FIF (Formaldehyd Induced Fluorescence) | 405 | 433 |

(continued)

| Fluorophore | excitation | emission |
|---|---|---|
| Flazo Orange | 375-530 | 612 |
| Fluoro-Gold (Hydroxystilbamidine) | 361 | 536 |
| Fura-2, high calcium | 335 | 505 |
| Fura-2, low calcium | 363 | 512 |
| GFP wild type, | 395 | 509 |
| GFPuv | 385 | 508 |
| Gloxalic Acid | 405 | 460 |
| Granular Blue | 355 | 425 |
| Hoechst 33258 | 345 | 487 |
| Hoechst 33342 | 347 | 483 |
| Hoechst 34580 | 392 | 440 |
| HPTS | 355 | 465 |
| Hydroxycoumarin | 325-360 | 386-455 |
| Hydroxystilbamidine (FluoroGold) | 361 | 536 |
| Hydroxytryptamine | 400 | 530 |
| Indo-1, high calcium | 330 | 401 |
| Indo-1, low calcium | 346 | 475 |
| Intrawhite Cf | 360 | 430 |
| Laurodan | 355 | 460 |
| Leucophor PAF | 370 | 430 |
| Leucophor SF | 380 | 465 |
| Leucophor WS | 395 | 465 |
| Lucifer Yellow | 425,428 | 528,536,540 |
| Lyso Tracker Blue | 373 | 422 |
| LysoSensor Blue | 374 | 424 |
| LysoSensor Yellow/Blue | 384 | 540 |
| Mag-Fura-2 Ratio Dye, Ca2+ | 369/329, 369/330 | 508, 511/491 |
| Mag-Fura-5 Ratio Dye, Mg2+ | 369/330, 369/332 | 505/500, 505/482 |
| Mag-Indo-1 | 349/328, 349/330 | 480/390, 480/417 |
| Marina Blue | 362 | 459 |
| Methoxycoumarin | 360 | 410 |
| Monobromobimane | 398 | 490 |
| Monobromobimane (mBBr-GSH) | 398 | 500 |
| Monochlorobimane | 380 | 461 |
| MPS (Methyl Green Pyronine Stilbene) | 364 | 395 |
| Noradrenaline | 340 | 490-520 |
| Nuclear Fast Red | 289-530 | 580 |
| Nuclear Yellow | 365 | 495 |

(continued)

| Fluorophore | excitation | emission |
|---|---|---|
| Pacific Blue | 405 | 455 |
| PBFI | 340/380 | 420 |
| Phorwite AR | 360 | 430 |
| Phorwite BKL | 370 | 430 |
| Phorwite Rev | 380 | 430 |
| Phorwite RPA | 375 | 430 |
| PhotoResist | 365 | 610 |
| PMIA | 341 | 376 |
| Primuline | 410 | 550 |
| Propidium Iodid (PI) | (305), 536, 538 | 617 |
| PyMPO | 412,415 | 561,564,570 |
| Pyrene | 360 | 387 |
| Pyronine | 410 | 540 |
| Pyrozal Brilliant Flavin 7GF | 365 | 495 |
| Sapphire GFP | 395 | 511 |
| SBFI | 340/380 | 420 |
| Serotonin | 365 520- | 540 |
| sgBFP™ | 387 | 450 |
| sgBFP™ (super glow BFP) | 387 | 450 |
| SITS | 336 | 436 |
| SITS (Primuline) | 395-425 | 450 |
| SITS (Stilbene Isothiosulphonic Acid) | 365 | 460 |
| SPQ (6-methoxy-N-(3-sulfopropyl) quinolinium) | 344 | 443 |
| Stilbene | 335 | 440 |
| Tetracycline | 390-425 | 525-560 |
| Thioflavin TCN | 350 | 460 |
| Thiolyte | 370-385 | 477-488 |
| Tinopol CBS (Calcofluor White) | 390 | 430 |
| True Blue | 365 | 425 |
| Uranine B | 420 | 520 |
| Uvitex SFC | 365 | 435 |
| wt GFP | 395 (475) | 508 |
| GFP Y66F | 360 | 508 |
| GFP Y66H | 360 | 442 |

Therefore compounds or the fluorescence part of the compounds of step b) of the inventive method are preferably derivatives of any fluorophore of table 1.

The compound of step b) may comprise a linking moiety or linker between the fluorophore or the fluorescence part and the part or subunit binding to or interacting with a thalidomide binding peptide.

The linking moiety or linker may be any suitable linker. It is important that the linker is not too long because the distance between the fluorophore and the binding part should be suitable to allow FRET (0.5 to 10 nm). The preferred distance between the fluorophore or the fluorescence part and the part or subunit binding to or interacting with a thalidomide binding peptide to be kept by the linker molecule corresponds to a C1 - C30 alkyl chain as a backbone of the linker molecule, more preferred to a C2 - C20 alkyl chain and particularly preferred to a C2 - C10 alkyl chain. The linking moiety or linker may be linear or branched as well as cyclic. Furthermore it is preferred if the linking moiety as well as the complete compound of step b) contains no or at least as few as possible quenching parts. Therefore the linking moiety should not comprise a nucleoside, acrylamide, and glycine.

Step b) of the inventive method relates preferably to a compound of at least two subunits or parts, one being a fluorophore having an excitation between 310 and 425 nm and the second subunit being able to bind to or interact with thalidomide binding peptide, optionally these subunits are thereby linked via linker moiety. The compound of step b) may therefore be a compound of general formula (I)

$$F\text{-}L\text{-}B \quad (I)$$

wherein F represents a Fluorophore, L represents a linking moiety and B is a structure capable of binding or interacting with the thalidomide binding domain of cereblon.

Using crystallography, ITC and NMR the inventors could already show that the following candidate substances and their derivatives bind to bacterial cereblon of M. *gryphiswaldense*: glutarimide, hydantoin, succinimide, oxazolidin-2,4-dione, uracil and 2-pyrrolidone.

Therefore preferred are compounds of step b) having an excitation frequency between 310 and 425 nm and bind to said thalidomide binding peptide wherein this binding is based on a molecule selected from the group comprising or consisting of glutarimide, hydantoin, succinimide, oxazolidin-2,4-dione, uracil and 2-pyrrolidone. Therefore these compounds or the binding part of these compounds of step b) of the inventive method are preferably derivatives of glutarimide, hydantoin, succinimide, oxazolidin-2,4-dione, uracil or $\gamma$-lactam.

Preferred are compounds based on uracil, wherein the subunit binding to said thalidomide binding peptide is uracil or uracil derivative. Further preferred are compounds based on glutarimide, wherein the subunit binding to said thalidomide binding peptide is glutarimide or glutarimide derivative.

The term "derivative" as used herein refers to a compound that is derived from a similar compound (like uracil) by some chemical or physical process. Furthermore it also refers to a compound that arises from another compound, if one atom is replaced with another atom or group of atoms. Hence is a structural analogue.

Crystallization experiments of the inventors show that uridine is a natural ligand of cereblon. Based on this knowledge the inventors generate the compound 1-(N- 2-MANT-imidoethyl)-uracil and used this compound for initially establishing the method of the present invention. It could be found that 1-(N-2-MANT-imidoethyl)-uracil is especially suitable for the method according to the present invention. Hence, one preferred embodiment of the invention is a method, wherein the compound of step b) is 1-(N-2-MANT-imidoethyl)-uracil.

The present application therefore refers also to the fluorescent compound 1-(N-2-MANT-imidoethyl)-uracil. 1-(N-2-MANT-imidoethyl)-uracil, called herein also MANT-uracil, has the following formula:

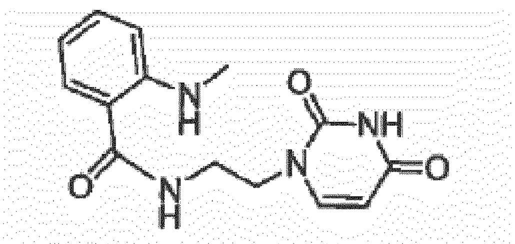

Chemical Formula: $C_{14}H_{16}N_4O_3$
Molecular Weight: 288,30

IUPAC nomenclature of 1-(N-2-MANT-imidoethyl)-uracil is as follows: *N*-[2-(2,4-Dioxo-3,4-dihydro-*2H*-pyrimidin-1-yl)-ethyl]-2-methylaminobenzamide.

Another aspect of the present invention is the use of 1-(N-2-MANT-imidoethyl)-uracil for identifying substances having a teratogenic potential. Especially preferred are methods according to the invention, wherein the compound

having an excitation frequency between 310 and 425 nm and a subunit binding to said thalidomide binding peptide is 1-(N-2-MANT-imidoethyl)-uracil.

Another preferred compound of step b) is a conjugate of uracil or glutarimide, ATTO 390 (ATTO-TEC GmbH), EDANS (5-((2-Aminoethyl)amino)naphthalene-1-sulfonic acid), Alexa Fluor® 350 (Life Technologies ), AMCA (aminomethylcoumarin acetate) and 5-HAT (5-hydroxy tryptamine) with excitation frequency between 370 and 415 nm and an emission range of 520 to 540 nm. This compound is especially suitable for methods of identifying binding to cereblon of the invention because the emission range of 520 to 540 nm has no overlap with the tryptophan fluorescence.

Step d) of the inventive methods refers to formation of a complex of the thalidomide binding peptide of step a) with the compound of step b). Forming a complex occurs by intermolecular forces, such as ionic bonds, hydrogen bonds and van der Waals forces. The docking has to be reversible. Step d) of the inventive methods could also be defined as mixing the thalidomide binding peptide of step a) with the compound of step b) and incubate this mixture. During incubating the complex of the thalidomide binding peptide of step a) with the compound of step b) takes place spontaneously, that means compound of step b) binds to the thalidomide binding peptide. Preferably step d) comprises incubation at room temperature for some minutes, approximately 10 minutes. Step d) as well as the complete method of the invention could be performed in solution. This means that the thalidomide binding peptide of step a) and the compound of step b) may be solved in one solution or that a solution of the thalidomide binding peptide is mixed with a solution of the compound of step b). Such a solution could be based on any suitable solvent as well as buffer solutions or a mixture of buffer and DMSO up to 0.03% (v/v). It should be avoided that the solvent has a quenching effect interfering with the inventive method and that the solvent or buffer denatures the peptide.

One preferred embodiment is a method wherein in step d) the thalidomide binding peptide of step a) is contacted or mixed with an equimolar amount of the compound of step b).

Step e) of the inventive methods comprises forming a reaction mixture by contacting the candidate substance with the complex of step d). Preferably the candidate substance or a solution of the candidate substance is added to the complex or to the solution of the complex of step d). A solvent for the candidate substance should not quench or interfere with a component of the inventive method. Denaturation of the peptide should be avoided, too. A reaction mixture of the inventive method has to include at least all three components, a thalidomide binding peptide, a compound binding to said thalidomide peptide and interacting with the tryptophan fluorescence of said thalidomide binding peptide, being preferably a compound having an excitation frequency between 310 and 425 nm, as well as a candidate substance. Furthermore there may be included a solvent or mixture of different solvents, a buffer system or optionally further additives like protease inhibitors.

One embodiment of the present invention refers to a method comprising step e) wherein different concentrations of the candidate substance are added to the complex of step d) (each concentration to one sample containing the complex of step d)) which allows a quantitative determination of the binding of the candidate substance after data analysis.

$IC_{50}$ and $K_i$ values for candidate substances can be determined as described in example 6 for thalidomide and example 7. Depending on the binding affinity it may be useful to determine an optimal concentration range for the assay in advance.

For control reasons, whether the method is actually working (in the hand of the actual user, under the conditions in the concrete laboratory) a negative probe and a positive reference sample should always be used together with the candidate substance to be tested. This is a standardized procedure with biologically or diagnostic assays. This means that at the same time or immediately one after the other not only the candidate substance is tested within the inventive method but also a negative control, which can be a blank and/or a sample containing a substance to be known not to bind to the thalidomide binding peptide. Such a blank would comprise all components, like thalidomide binding peptide, compound of step b) and optionally buffer, except for the candidate substance.

Step f) of the inventive methods refers to incubating the reaction mixture under conditions sufficient to allow the candidate substance to replace the compound of step b) in the complex of step d), in case the candidate substance is able to bind to the thalidomide binding peptide.

This means that the reaction mixture is incubated for some time, preferably between 2 and 30 minutes, more preferred between 10 and 20 minutes. The term "suitable conditions" refers mostly to a suitable temperature, preferably at room temperature, and a suitable solvent or buffer used in the reaction mixture. Suitable buffer solutions should not react with one of the components of the inventive method (assay) and should not precipitate. Furthermore a suitable buffer should not have own fluorescence. Suitable are reaction mixtures on basis of PBS-buffers (Phosphate buffered saline) as well as Tris- and triethanolamine buffer. It is preferred if the pH-value of the used buffer is higher than the isoelectric point of the used thalidomide binding protein. Therefore it is preferred that the used buffer has a pH $\geq$ 6.5. Suitable buffers should not contain or only contain minor amounts of fluorescence quenching substances like DMSO, acrylamide, iodine ions, and glycine.

Step g) of the inventive methods comprises determining if the compound of step b) has been replaced in the complex of step d) by the candidate substance. This is preferably done by photometry. In a reaction mixture containing a candidate substance and a complex of the thalidomide binding peptide with the compound of step b) the candidate

substance and the compounds of step b) compete for the binding site at the thalidomide binding peptide. If the candidate substance is able to specifically bind cereblon and therefore to bind the thalidomide binding peptide of the inventive method, a part of the thalidomide binding peptide releases the compound of step b) and binds to the candidate substance to be tested. Because the principle of fluorescence quenching needs spatial proximity replacement of compound of step b) in the complex leads to a decrease in fluorescence quenching by the compound of step b), a fact which can be measured. In general this effect may be measured at the emission maximum of the tryptophan residues of the thalidomide binding peptide, which is at 375 nm (cf. Figure 3). Therefore determining if the compound of step b) has been replaced in the complex by the candidate substance may comprise measuring the fluorescence in a range of 350 nm - 380 nm. A decrease in fluorescence quenching is thereby indicative for binding of the candidate substance to the thalidomide binding peptide and therefore the candidate substance is assumed to be teratogenic. However in the case that the assay is based on FRET, a decrease of fluorescence of the compound of step b) would be indicative for teratogenicity of the candidate substance. In both cases the method of the present invention enables the detection of binding of a test substance to a thalidomide binding peptide, like cereblon, with a cost-effective optical or photometrical measurement. The direct optical detection method allows further for analyzing the binding affinity and has the advantage that the method of the invention is suitable for miniaturizing and automatization.

The specific arrangement of three tryptophan residues, conserved in cereblon of all species and in all cereblon-homologous proteins, surrounding the candidate substance bound to the binding pocket of the thalidomide binding peptide, allows using the FRET effect to determine and characterize binding of the candidate substances to cereblon. Then the compound of step b) should have an excitation frequency between 310 and 425 nm.

In the complex of step d) the excitation energy of the specifically excited tryptophan residues in the binding pocket may be directly transferred to said compound of step b), more precisely to the part of said compound having an excitation frequency between 310 and 425 nm. The thereby excited compound of step b) will emit light at a specific wavelength in turn (FRET effect).

If a reaction mixture containing a candidate substance is added to the complex of the thalidomide binding peptide and a compound of step b) having an excitation frequency between 310 and 425 nm, the candidate substance and the compounds having an excitation between 310 and 425 nm and binding to a thalidomide binding peptide compete for the binding site at the thalidomide binding peptide.

If the candidate substance is able to specifically bind cereblon and therefore to bind the thalidomide binding peptide of the inventive method based on FRET, a part of the thalidomide binding peptide releases the compound of step b) and binds to the candidate substance to be tested. Because the FRET effect and hence the excitation of the compound of step b) needs spatial proximity, replacement of compound of step b) leads to a decrease in the fluorescence of the compound of step b), a fact which can be spectroscopically measured.

In a preferred embodiment of the method or assay according to the present invention FRET could take place (if the candidate substance binds or interacts with cereblon) between the fluorescence of the excited tryptophan residues in the binding pocket (excitation: 295 nm) and the fluorophore or fluorescent part of the compound of step b) (for MANT-uracil excitation: 350 nm, emission: 440 nm). Thus, the inventive methods are preferably based on the general principle of establishing a connection between the reduction of the fluorescence of the compound of step b) and the binding of the candidate substance to the thalidomide binding peptide which indicates a teratogenic potential.

Step g) of the preferred embodiment of the inventive method based on FRET refers therefore to determining a fluorescence decrease at a specific wave length, namely the emission maximum of the compound of step b), which occurs when the candidate substance is added and replaces the compound of step b) in the complex. A decrease in fluorescence indicates a binding of the candidate substance to the thalidomide binding peptide. Determining a decrease in fluorescence requires two steps, excitation at an excitation wavelength of the tryptophan residues which not excites or only minimally excites compound b) (preferably at 295 nm) and subsequently measurement of the emission at the emission wavelength of compound b) (preferably at 440nm if compound b) is 1-(N-2-MANT-imidoethyl)-uracil) which is distinguishable from the emission of the tryptophan residues of the thalidomide binding peptide. It is common knowledge that determining a fluorescence decrease or increase is only possible if a blank sample and/or a sample containing a substance to be known not to bind are measured, too.

Therefore step g) comprises preferably comparison of the measured emission with a blank value and/or the value of at least one reference probe. The emission is preferably measured somewhere in the range of more than 430 nm and most preferably measured at 440nm (for if compound b) is 1-(N-2-MANT-imidoethyl)-uracil) or at 520-540nm (if compound b) is based on 5-HAT). Nevertheless the optimum may shift depending on the used compound of step b). Within an embodiment based on FRET the wavelength used to determine if binding occurs should be clearly separated from the emission frequency of the tryptophan emission frequency of the thalidomide binding peptide in the used buffer system alone. Furthermore it is also possible to measure the decrease in donor fluorescence (tryptophan residues) instead of the increase in acceptor fluorescence, here compound of step b) (Quenched FRET-assay).

If different concentrations of the candidate substance are tested within the method of the present application it allows a quantitative measurement of the binding of the candidate substance after data analysis. Using an exponential

fit of the values for the measured emission it is possible to determine the saturation ($B_{max}$) of a binding curve. The crystal structure (see figure 1) has shown that the stoichiometry of the binding is 1:1. Therefore the emission values may be converted into a relative amount of bound candidate substance.

In order to exclude or to minimize false-negative results within method of identifying substances which bind to a thalidomide binding peptide of the invention it is recommended to determine if the test substance interacts with the thalidomide binding peptide in a way which has influence to the result of the assay.

If step g) and therefore determination if the compound of step b) has been replaced in the complex by the candidate substance is based measuring a FRET effect in the binding pocket it is recommended to test that the candidate substance itself does not emit fluorescence at the same wavelength the compound of step b) does and which is the wavelength FRET effect is measured. This can be done by measuring the excitation and emission spectra of the candidate substance, preferably in the assay buffer, before starting with the assay. The candidate substance should not be excitable by tryptophan fluorescence or in step g) determining the possible replacement should comprise measurement of the emission of the compound of step b) at a wavelength the candidate substance does not emit fluorescence. In the very rare case that there is a full overlap of excitation and emission wavelength of the compound of step b) and the candidate substance it is possible to use another compound according to step b) having another fluorophore without an overlap of excitation or emission spectra (see table 1).

If step g), and therefore determination if the compound of step b) has been replaced in the complex by the candidate substance, is based on evaluating the quenching effect by measuring directly the intensity of the fluorescence emission of the tryptophan residues in the binding pocket it is recommended to test that the candidate substance itself does not quench the tryptophan fluorescence, too.

It is possible to test for a possible quenching effect of the candidate substance on the tryptophan fluorescence of the thalidomide binding peptide by measuring in parallel and subsequently compare the emission for the following three reaction mixtures:

1. Mixture containing the thalidomide binding peptide and one of several concentrations of the candidate substance, preferably in assay buffer;
2. Mixture containing one of several concentrations of the candidate substance only, preferably in assay buffer,
3. Mixture containing the thalidomide binding peptide, a compound of step b) being a suitable FRET partner for the thalidomide binding peptide (like MANT-uracil) and one of several concentrations of candidate substance, preferably in assay buffer.

If the emission of the tryptophan residues of the thalidomide peptide (around 350 nm) obtained from mixture 1 with several concentrations of the candidate substance plotted against the candidate substance concentration shows the characteristics of a competitive binding curve, the substance obviously quenches the fluorescence of the tryptophan residues in the binding pocket. This may be verified by the result of mixture 3. If FRET occurs the candidate substance binds to the binding pocket of the thalidomide binding peptide.

Therefore the method of identifying substances which bind to a thalidomide binding peptide of the invention may further comprise a step referring to a test for a possible quenching effect of the candidate substance. Thus said method may further comprise step h) verification that the candidate substance does not interact with the tryptophan fluorescence of the thalidomide binding peptide in a way influencing the result of step g).

Thus one embodiment of the invention refers to a method of identifying substances which bind to a thalidomide binding peptide comprising the following steps:

a) providing a thalidomide binding peptide
b) providing a compound binding to said thalidomide binding peptide interacting with the tryptophan fluorescence of the thalidomide binding peptide
c) providing a candidate substance to be tested
d) forming a complex of the thalidomide binding peptide with the compound of step b)
e) forming a reaction mixture by contacting the candidate substance with the complex of step d)
f) incubating the reaction mixture under conditions sufficient to allow the candidate substance to replace the compound of step b) in the complex of step d), in case the candidate substance is able to bind to the thalidomide binding peptide,
g) determining if the compound of step b) has been replaced in the complex of step d) by the candidate substance
h) verification that the candidate substance does not interact with the tryptophan fluorescence of the thalidomide binding peptide in a way influencing the result of step g)

Another embodiment of the present invention relates to a method of identifying substances which bind to cereblon comprising or consisting of the following steps:

a) providing a thalidomide binding peptide
b) providing a compound having an excitation frequency between 310 and 425 nm and binding to said thalidomide binding peptide
c) providing a candidate substance to be tested
d) forming a complex of the thalidomide binding peptide with the compound of step b)
e) forming a reaction mixture by contacting the candidate substance with the complex of step d)
f) incubating the reaction mixture under conditions sufficient to allow the candidate substance to replace the compound of step b) in the complex of step d), in case the candidate substance is able to bind to the thalidomide binding peptide,
g) determining if the compound of step b) has been replaced in the complex of step d) by the candidate substance by measuring the emission of the reaction mixture at the emission frequency of the compound of step b) and subsequently comparing of the measured emission with value of a blank and/or of at least one reference probe.

Preferred are further methods wherein the candidate substance does not quench the tryptophan fluorescence of the thalidomide binding peptide and in particular preferred are methods wherein the test substance does not emit light at the same wave length than the compound of step b) if the compound of step b) contains a fluorophore or a fluorescence part and the assay is based on the principle of FRET.

Another embodiment of the present invention is a kit for identifying test compounds having a teratogenic potential containing a) a thalidomide binding peptide, preferably cereblon protein or the thalidomide-binding domain of cereblon or a homologue thereof and b) a compound binding to said thalidomide binding peptide interacting with the tryptophan fluorescence of the thalidomide binding pocket, wherein compounds having an emission between 310 - 425 nm and binding to said thalidomide binding peptide are preferred.

A kit in molecular biology or in medical diagnostics is a package which includes all necessary ingredients for performing a certain method or singular step. Standard chemicals as present in any standard molecular biology, chemical or medical laboratory are normally not included. Nevertheless some of these standard chemicals may be indispensable to carry out the binding assay or the method properly. It is understood that all ingredients are provided in quantities that allow for a proper execution of the desired reactions for the majority of scientific, diagnostic and industrial applications.

Often, but not always, these ingredients are provided in already prepared solutions ready- or close to ready-for-use. There may be also combinations of different ingredients already added together. A further advantage is that such kits use to be verified. Therefore the operator doesn't have to prove again the viability of the diagnostic method and can save on at least some control experiments. Therefore kits are a very popular tool in laboratories in research, diagnostics and industry.

The following components may also be included in such kits:

c) wash solution
d) sample buffer and or reaction buffer
e) microtiter plate suitable for fluorescence measurement
f) stock solution of a reference sample

**Description of the Figures**

Figure 1: **A:** Three-dimensional crystal structure of cereblon homologue of *M. gryphiswaldense* (GI:1448978003) in complex with thalidomide. **B:** Spatial arrangement of the bound thalidomide within the binding pocket.

Figure 2: Spectrum of the fluorescence emission (excitation at 295 nm) of cereblon homologue *M. gryphiswaldense* wild type (MgCRBN WT), the W36/59F mutant thereof (MgCRBN W36/59F), MANT-uracil and MgCRBN W36/59F in complex with MANT-uracil (10 μM each).

Figure 3: Fluorescence emission spectrum (excitation at 295 nm) of MgCRBN W36/59F during titration with increasing concentration of MANT-uracil (0.2 - 44.2 μM). The continuous line represents the emission of MgCRBN W36/59F without MANT-uracil.

Figure 4: **A:** DMSO blank determination; fluorescence at 440 nm within the inventive method using MgCRBN W36/59F and MANT-uracil. No candidate substance has been added but buffer with increasing amounts of DMSO to determine a possible quenching effect of DMSO
**B:** Fluorescence at 440 nm within the inventive method using MgCRBN W36/59F and MANT-uracil and

adding increasing amount of thalidomide.

Figure 5: Percentaged presentation of the concentration - depended inhibition of the binding of MANT-uracil to MgCRBN W36/59F in presence of thalidomide.

Figure 6: **A:** Fluorescence emission spectrum (excitation at 295 nm) of MgCRBN W36/59F during titration with increasing concentration of MANT-uracil (0 - 35 μM). The continuous line represents the exponential fit to obtain $B_{max}$.
**B:** Converted FRET data from Fig.6A shown as a plot of bound MANT-uracil fraction against free MANT-uracil.

Figure 7: Comparison of the binding modes of thalidomide, lenalidomide, pomalidomide, deoxyuridine, hydantoin, glutarimide, ethosuximide and aminoglutethimide in the respective crystal structures. The orientation of the binding pocket in the individual panels is the same as in Figure1B. In all cases the imide or the imide moiety of the compound is bound in the same orientation, making the same contacts to the protein as thalidomide in Figure 1. The central panel shows a superposition of all compounds onto the thalidomide-bound binding pocket.

Figure 8: The diagram shows bound MANT-uracil concentration plotted against free MANT-uracil concentration during the titration of mutant W36/59F CRBN homologue from *M. gryphiswaldense* with increasing concentration of uridine. The data were converted from FRET data measured at 440 nm after excitation at 295 nm as described.

Figure 9: **A:** The diagram shows bound MANT-uracil concentration plotted against free MANT-uracil concentration during the titration of *C. elegans* CRBN with increasing concentration of MANT-uracil. The data were converted from FRET data measured at 440 nm after excitation at 295 nm as described. **B:** Fluorescence at 440 nm within the inventive method using *C. elegans* CRBN-homologue and MANT-uracil and adding increasing amount of thalidomide.

Figure 10: Scheme showing the nomination of vectors and angles between of an exemplary unit cell.

The following abbreviations are used for the common and modified amino acids referred to herein:

| 3 letter code | One letter code | Amino acid |
|---|---|---|
| Ala | A | Alanine |
| Arg | R | Arginine |
| Asn | N | Asparagine |
| Asp | D | Aspartic acid (Aspartate) |
| Cys | C | Cysteine |
| Gln | Q | Glutamine |
| Glu | E | Glutamic acid (Glutamate) |
| Gly | G | Glycine |
| His | H | Histidine |
| Ile | I | Isoleucine |
| Leu | L | Leucine |
| Lys | K | Lysine |
| Met | M | Methionine |
| Phe | F | Phenylalanine |
| Pro | P | Proline |
| Ser | S | Serine |

(continued)

| 3 letter code | One letter code | Amino acid |
|---|---|---|
| Thr | T | Threonine |
| Trp | Y | Tryptophan |
| Tyr | W | Tyrosine |
| Val | V | Valine |

## Examples

### <u>Example 1: Cloning and PCR-Mutagenesis of CRBN homologue from *M. gryphiswaldense* (GI:144897803)</u>

For expression of the CRBN homologue from *M. gryphiswaldense,* a DNA fragment which was codon optimized for expression in *Escherichia coli* was used. The according DNA fragment was synthesized and cloned in vector pUC57 by Genscript. For heterologous expression it was subcloned in the NcoI and BamHI sites of the vector pETHis_1a. After verification of the sequence by DNA sequencing, the plasmid was transformed in *E. coli* C41 cells for expression. In the mutant W36/59F variant of the CRBN homologue from *M. gryphiswaldense* the surface located tryptophan residues in position 36 and 59 of the sequence of the CRBN homologue from *M. gryphiswaldense* were exchanged by PCR mutagenesis using the QuikChange® Site-Directed Mutagenesis Kit from Stratagene (now an Agilent Technologies company; Santa Clara, California, USA). A codon optimized sequence of the CRBN homologue from *M. gryphiswaldense* cloned in vector pETHis_1a was used as a template. Mutagenesis primers were as followed:

W36F se 5'-CCATTAGCCGTCGCGATTTCCTGCTGCCGATGGGCGGTGA

W36F as 5'-TCACCGCCCATCGGCAGCAGGAAATCGCGACGGCTAATGG

W59F se 5'-GGCATGATTTTTCGTGTCTTCTGCTTCAGCCTGGCACAG and

W59F as 5'-CTGTGCCAGGCTGAAGCAGAAGACACGAAAAATCATGCC.

For construction of the mutant MgCRBN variant W36/59F, Y101 F primers

Y101 F se 5'-GTCATCTGGGTTGGCACTTCGAAGGCGGTTCCCAACCG,

Y101 F as 5'-CGGTTGGGAACCGCCTTCGAAGTGCCAACCCAGATGAC

and DNA of variant W36/59F as a template were used.

Mutagenesis was performed according to the manual of the manufacturer. The correctness of the sequences was proofed by sequencing the appropriate clones using T7 standard primers (forward and reverse).

### <u>Example 2: Expression and Isolation of the CRBN homologue from *M. gryphiswaldense* and its mutant variants W36/59F and W36/59F, Y101F of example 1</u>

For expression of the CRBN homologue from *M. gryphiswaldense,* a DNA fragment which was codon optimized for expression in Escherichia coli was used. The according DNA fragment was synthesized and cloned in vector pUC57 by Genscript. For heterologous expression it was subcloned in the NcoI and BamHI sites of the vector pETHis_1a. After verification of the sequence by DNA sequencing, the plasmid was transformed in *E. coli* C41 cells for expression. Cells were grown at 37°C in the presence of kanamycin up to an optical density of 0.5 measured at 600 nm. Protein expression was induced by addition of a final concentration of 1 mM IPTG to the culture. After incubation for another 4 hours, cells were harvested by centrifugation. The cell pellet was resuspended in 20 mM Tris/HCl, pH 7.5, 150 mM NaCl, 4 mM $MgCl_2$, 5 mM β-mercaptoethanol with addition of DNase, phenyl methane sulfonylfluoride and protease inhibitor mix (Roche, Basel). The cells were disrupted using a French pressure cell. After centrifugation of the extract, the supernatant was loaded on a Ni-NTA column equilibrated with 20 mM Tris/HCl, pH 7.9, 300 mM NaCl, 5 mM β-mercaptoethanol. The column was washed with 1.5 column volumes of 25 mM imidazole in equilibration buffer followed by another 1.5 column volumes of 50 mM imidazole containing buffer. The bound CRBN homologue was eluted using a linear gradient

of 0.05-0.5 M imidazole in 20 mM Tris/HCl, pH 7.9, 500 mM NaCl, 5 mM β-mercaptoethanol. The fractions containing the CRBN homologue of *M. gryphiswaldense* were pooled and dialyzed against 20 mM Tris/HCl, pH 7.5, 150 mM NaCl, 5 mM β-mercaptoethanol. The N-terminal histidine tag was cleaved by incubation with TEV protease at 4°C over night. The cleaved linker and the His-tagged TEV protease were removed by a Ni-NTA column using 20 mM Tris/HCl, pH 7.5, 150 mM NaCl, 5 mM β-mercaptoethanol as loading buffer. Bound proteins were eluted using a 0-0.5 M imidazole gradient. The untagged CRBN-homologue was found partly in the flow-through, but eluted mainly at about 0.02 mM imidazole from the column. The protein fractions were pooled and dialyzed over night against 20 mM Tris/HCl, pH 7.5, 150 mM NaCl, 5 mM β-mercaptoethanol and concentrated using Amicon Ultra-15 Centrifugal Filter Units.

Mutant protein variants W36/59F and W36/59F, Y101 F were overexpressed and purified as described for wild type CRBN of *M. gryphiswaldense.* Due to cloning requirements and leftovers after protease cleavage, each of the CRBN of *M. gryphiswaldense* variant proteins described here contains additional amino acid residues at their N-terminus. The resulting N-terminus is Gly-Ala-Met-Gly followed by the original sequence starting with Pro in position 2 (SEQ ID No. 3 and 6).

**<u>Example 3:</u> Crystallization and Crystal Structure Determination of the cereblon homologue of *M. gryphiswaldense* in complex with thalidomide**

Crystals were obtained via the vapor diffusion method using 96well sitting drop crystallisation plates. The wild-type protein solution prepared in Example 2 was concentrated to 10 mg/ml and supplemented with thalidomide, using a 100mM thalidomide stock solution in 100% DMSO, to a final concentration of 1mM thalidomide and 1% DMSO. The reservoir solution contained 100 mM magnesium chloride, 100mM sodium citrate pH 5.0, and 15%(w/v) PEG 4000. In the crystallization setup, 400 nl of the protein:thalidomide solution were mixed with 400 nl of reservoir solution and equilibrated against 75 μl reservoir solution at 20°C. Crystals grew within 3 days, were transferred into a cryo-solution containing 20%(v/v) PEG 400 in addition to the reservoir solution for several seconds, loop mounted, and flash frozen in liquid nitrogen. Data were collected under cryo conditions at 100K at beamline X10SA of the Swiss Light Source (SLS,PSI,Villigen,CH) with a wavelength of 1Å on a PILATUS detector (DECTRIS). Data were processed to a resolution of 1.4Å using XDS (Kabsch, 1993). Exploiting the anomalous contribution of the structural Zinc ion bound to the protein, the structure was solved via single anomalous dispersion (SAD) using SHELXD/E (Sheldrick, 2008). The maps output by SHELXE after density modification were used to build an initial model with ARP/WARP (Perrakis et al., 1999). The structure was completed in cyclic manual modeling with Coot (Emsley and Cowtan, 2004) and refinement with REFMAC (Murshudov et al., 1999). The crystals contain 3 monomers in the asymmetric unit in space group P212121, which are all in complex with Thalidomide. The structure of one monomer is shown in Figure 1. Data collection and refinement statistics are summarized in Table 2. The atomic coordinates are shown in Figure 10. The following is a list of different reservoir solutions tested and found to be adequate within the above described protocol for growing crystals of the cereblon homologue from *M. gryphiswaldense* in complex with thalidomide or a related ligand of sufficient quality for structure determination:

0.1 M tri-Sodium citrate pH 5.6 1.0 M Ammonium phosphate
0.1 M Citric acid pH 3.5 25 %(w/v) PEG 3350
0.1 M Sodium acetate pH 4.5 25 %(w/v) PEG 3350
0.2 M L-Proline 0.1 M HEPES pH 7.5 10 %(w/v) PEG 3350
0.064 M Sodium citrate pH 7.0 0.1 M HEPES pH 7.0 10 %(w/v) PEG 5000 MME
0.1 M Sodium acetate pH 4.6 25 %(w/v) PEG 1000
0.1 M Sodium acetate pH 4.6 25 %(w/v) PEG 8000
0.1 M Sodium acetate pH 4.6 20 %(w/v) PEG 10000
0.1 M Sodium acetate pH 4.6 15 %(w/v) PEG 20000
0.1 M Sodium HEPES pH 7.5 15 %(w/v) PEG 20000
0.1 M HEPES pH 7.5 20 %(w/v) PEG 1500
0.1 M tri-Sodium citrate pH 5.6 10 %(w/v) PEG 4000 10 %(w/v) Isopropanol
0.2 M Ammonium sulfate 0.1 M Sodium acetate pH 4.6 12 %(w/v) PEG 4000
15 %(w/v) PEG 6000 5 %(w/v) Glycerol
0.1 M HEPES pH 7 15%(w/v) PEG 4000
0.1 M HEPES pH 7 10%(w/v) PEG 4000 10% (v/v) Isopropanol
0.1 M Magnesium chloride 0.1 M Sodium citrate pH 5 15%(w/v) PEG 4000
0.1 M Sodium citrate pH 4.5 20%(w/v) PEG 4000
0.1 M Potassium chloride 0.1 M HEPES pH 7.5 15%(w/v) PEG 6000
0.1 M MES pH 6.5 10%(w/v) PEG 5000 MME 12% (v/v) 1-propanol
0.1 M HEPES pH 7 15%(w/v) PEG 20000
0.2 M Ammonium chloride 20 %(w/v) PEG 3350
0.1 M Citric acid pH 4.0 20 %(w/v) PEG 6000 5.0

0.2 M Sodium chloride 0.1 M Phosphate-citrate pH 4.2 20 %(w/v) PEG 8000
0.1 M SPG buffer pH 9 25% w/v PEG 1500
0.2M Ammonium chloride 0.1 M Sodium acetate pH 5 20% w/v PEG 6000
0.1 M MMT buffer pH 4 25% w/v PEG 1500
0.1 M MMT buffer pH 6 25% w/v PEG 1500
0.2M Potassium thiocyanate 20% w/v PEG 3350
0.08 M TRIS.HCl pH 8.5 1.6 M Ammonium phosphate 20 %(v/v) Glycerol
0.6 M Sodium phosphate 0.6 M Potassium phosphate 0.075 M HEPES sodium salt pH 7.5 25 %(v/v) Glycerol
0.085 M HEPES pH 7.5 8.5 %(w/v) PEG 8000 15 %(v/v) Glycerol
1.0 M Sodium malonate 5.0
0.1 M MES pH 6.5 0.3 M Sodium fluoride
0.1 M Sodium acetate pH 4.6 0.3 M Sodium fluoride
0.1 M Sodium acetate pH 4.6 2.2 M Calcium chloride
0.1 M Citric acid pH 4.0 0.8 M Ammonium sulfate

**Example 4: Structure determination of the cereblon homologue of *M. gryphiswaldense* in complex with other compounds**

Additionally, crystal structures were solved with the protein in complex to lenalidomide, pomalidomide, deoxyuridine, hydantoin, glutarimide, ethosuximide, and aminoglutethimide. 100 mM stock solutions were prepared for all compounds; for lenalidomide and pomalidomide in 100% DMSO, for aminoglutethimide in 100% acetonitrile, for all others in pure water. The structures in complex with lenalidomide, pomalidomide and deoxyuridine were obtained by co-crystallization as described in Example 3. Here the protein solution was supplemented with 1mM of the ligand from the respective stock solution. Crystals grew under similar conditions as described for thalidomide and listed in Example 3, and the crystals were treated and data collected as described in Example 3. The crystals had essentially the same unit cell dimensions and space group as the crystals in Example 3. The structures were solved on the basis of the structure in complex with thalidomide in Example 3 and underwent the same modeling and refinement procedure as described in Example 3. In all cases, the 3 monomers in the asymmetric unit are in complex with the ligand added for co-crystallization, lenalidomide, pomalidomide or deoxyuridine, respectively. The structures in complex with hydantoin, glutarimide, ethosuximide, and aminoglutethimide were obtained by soaking experiments. Here, the crystals in complex with thalidomide from Example 3 were soaked in reservoir solution supplemented with 5mM ligand from the respective stock solution for 24h. Subsequently, the crystals were cryoprotected as in Example 3 and structure solution was done as described for lenalidomide, pomalidomide and deoxyuridine, on the basis of the structure in complex with thalidomide. Due to the soaking procedure, individual monomers in the unit cells had their ligand (thalidomide) exchanged to the ligand present in the soaking solution. For aminoglutethimide one, for hydantoin and ethosuximide two, and for glutarimide all three monomers in the asymmetric unit were in complex with the respective ligand. The binding modes of all 7 compounds in the 7 structures described here and of thalidomide in Example 3 are shown and compared in Figure 7. Data collection and refinement statistics are summarized in Table 2.

Table 2: Data collection and refinement statistics of the crystal structures of the cereblon homologue of *M. gryphiswaldense* in complex with thalidomide and other ligands. Values in parentheses refer to the highest resolution shell.

| Compound | Thalidomide | Lenalidomide | Pomalidomide | Deoxyuridine | Hydantoin | Glutarimide | Ethosuximide | Aminoglutethimide |
|---|---|---|---|---|---|---|---|---|
| a (Å) | 56.5 | 56.7 | 56.7 | 56.8 | 56.4 | 56.1 | 56.9 | 56.8 |
| b (Å) | 60.1 | 59.8 | 60.5 | 60.0 | 58.5 | 60.0 | 59.8 | 60.5 |
| c (Å) | 88.5 | 88.1 | 88.7 | 88.4 | 87.1 | 88.1 | 88.1 | 88.5 |
| Resolution (Å) | 40 - 1.40 (1.48-1.40) | 40 - 1.85 (1.96 - 1.85) | 40 - 1.55 (1.64 - 1.55) | 40 - 1.80 (1.91 - 1.80) | 40 - 1.85 (1.96 - 1.85) | 40 - 2.50 (1.65 - 2.50) | 40 - 2.10 (2.22 - 2.10) | 40 - 1.70 (1.80 - 1.70) |
| Completeness (%) | 99.5 (97) | 98.7 (97.6) | 99.0 (95.7) | 98.4 (95.9) | 97.8 (94.6) | 96.9 (94.6) | 99.2 (97.1) | 98.8 (98.6) |
| Redundancy | 6.7 (6.4) | 2.9 (2.8) | 3.8 (3.5) | 2.9 (2.7) | 2.9 (2.9) | 2.3 (2.3) | 3.4 (3.2) | 2.9 (2.9) |
| $I/\sigma(I)$ | 12.4 (1.74) | 8.0 (1.87) | 12.6 (1.91) | 8.4 (2.12) | 12.6 (1.86) | 6.55 (1.70) | 13.4 (2.3) | 14.4 (1.86) |
| $R_{merge}$ (%) | 7.4 (72.9) | 7.7 (43.0) | 5.2 (49.0) | 8.5 (53.9) | 6.1 (59.5) | 11.0 (49.0) | 6.5 (48.6) | 4.1 (56.6) |
| $R_{cryst}$ (%) | 16.4 | 22.4 | 17.5 | 16.0 | 17.0 | 20.7 | 17.5 | 16.9 |
| $R_{free}$ (%) | 20.0 | 27.5 | 21.6 | 21.9 | 21.6 | 27.5 | 22.9 | 22.0 |

It could be shown that the conformation of the cereblon homologue of M. *gryphiswaldense* stays identical after exchange of ligands. Therefore the structure in Figure 1 is representative for all evaluated complexes of the cereblon homologue and its ligands.

**Example 5: Determination of the equilibrium constant of MANT-Uracil**

Black 96-well plates were measured on a Biotek Synergy H4 Hybrid Multi-Mode microplate reader or a Biotek Synergy MX microplate reader using the following settings: top excitation and collection, excitation: 295 ± 9 nm, emission collection: 440 ± 9 nm).

A stock solution of MANT-Uracil was prepared at a concentration of 10 mM in DMSO. The influence of quenching caused by DMSO was verified by pipetting increasing concentrations of DMSO (0-0.1% v/v) in 110 μl of 3 μM CRBN homologue mutant W36/59F from *M. gryphiswaldense* in assay buffer (20 mM Tris/HCl, pH 7.5, 150 mM NaCl, 0.5 mM β-mercaptoethanol). Quenching effects were evaluated by recording fluorescence emission at 440 nm (excitation 295 nm). For concentrations up to 0.03 % DMSO no influence on emission at 440 nm was observed (Figure 4A).

Binding of MANT-Uracil to the cereblon homologue W36/59F mutant of *M. gryphiswaldense* was measured by dispensing increasing volumes (1-8 μl) of several dilutions of the MANT-uracil stock solution in 110 μl of 3 μM W36/59F mutant in the assay buffer. Several stock solutions of MANT-uracil (10 μM, 100 μM and 1 mM) were prepared by diluting the 10 mM MANT-uracil stock solution in assay buffer. The final concentrations of single measuring points were 0 μM, 0.09 μM, 0.18 μM, 0.265 μM, 0.35 μM, 0.435 μM, 0.52 μM, 0.6 μM, 0.68 μM, 0.76 μM, 0.9 μM, 1.345 μM, 1.79 μM, 2.22 μM, 2.65 μM, 3.08 μM, 3.51 μM, 3.93 μM, 4.35 μM, 5.17 μM, 5.98 μM, 6.78 μM, 17.86 μM, 26.55 μM, and 35.1 μM. Three dilution series for each MANT-uracil concentration were pipetted independently. Samples were excited at 295 nm and fluorescence emission was measured at 440 nm.

As a blank data set, adequate dilutions of DMSO in buffer were dispensed with 3 μM protein in assay buffer and measured under the same conditions. A second reference was measured by titration of the appropriate concentrations of MANT-uracil in buffer resulting in a concentration-dependent, linear increase of fluorescence emission at 440 nm. Binding analysis and blank data were analyzed by calculating mean values and standard deviation of the three measured data points for each concentration. After subtraction of the references, the obtained FRET data resemble the pool of MANT-uracil bound to protein. Fig. 6A shows a typical binding kinetic describing the dependency of the FRET signal on the total concentration of MANT-uracil. Performing an exponential plot of the binding curve, we determined the maximum of the emission at 440 nm with $B_{max}$ = 13336.34. This value corresponds to the saturation of the binding pocket of the cereblon homologue by bound ligand. Based on the binding stoichiometry (ligand to protein) of 1:1 obtained from the crystal structure, these data were converted in the relative amount of bound MANT-uracil (BL) which corresponds to 3 μM at $B_{max}$. The obtained data were subsequently subtracted for each measuring point in order to obtain the relative concentration of free MANT-uracil (FL). The plot axis were rescaled for bound versus free MANT-uracil and fitted with a hyperbola using the parameter for ligand binding with one binding pocket (Fig. 6B):

$$[BL] = \frac{B_{max}[FL]}{K_d + [FL]}$$

Fitting with $B_{max}$ = 3 μM which corresponds to the maximum of bound ligand resulted in a $K_d$ =1.13 ± 0.2 μM.

**Example 6: Determination of $IC_{50}$ values for thalidomide using the inventive method**

The same experimental conditions as described for example 5 were used for determination of the $IC_{50}$ value of thalidomide binding to the W36/59F mutant of the cereblon homologue from *M. gryphiswaldense.*

A 30 μM protein solution was preincubated with equimolar amounts of MANT-uracil in assay buffer for 5-10 min at 25°C. This mixture of protein with MANT-uracil was titrated with increasing concentrations of thalidomide. This was done in a final volume of 100 μl per well of a 96 well plate with final concentrations of 3 μM mutant cereblon homologue, 3 μM MANT-uracil and thalidomide concentrations of 1 μM, 2 μM, 4 μM, 5 μM, 7.5 μm, 10 μM, 15 μM and 30 μM. Thalidomide was diluted in assay buffer from a 100 mM stock solution in DMSO. As described for example 5 and shown in Fig. 4A, further addition of DMSO in amounts up to 0.03% does not influence fluorescence emission at 440 nm. Accordingly, thalidomide diluted in buffer can be added unconsidered up to a final concentration of 30 μM in the assay. Data sets containing three data points were measured for each concentration. Mean values and standard deviation were calculated as described. The measurement of the different thalidomide concentrations in buffer served as references.

Increasing thalidomide concentrations reduce the FRET effect and hence the emission at 440 nm of bound

MANT-uracil. Fig. 4B shows the FRET data plotted against the total concentration of thalidomide and fitted using the following equation of the standard four parameter logistic curve fit:

$$y = \min + \frac{(\max - \min)}{1 + (x/IC50)^{-Hillslope}} .$$

The obtained $IC_{50}$ value for thalidomide is $IC_{50}$ = 3,81 ± 0,48 μM.

**Example 7: Determination of $K_d$, $IC_{50}$ and $K_i$ values for candidate substances using the inventive method**

$IC_{50}$ values for candidate substances can be determined as described in example 6 for thalidomide. Depending on the binding affinity it appears useful to determine the optimal concentration range for the assay in advance. It is further recommended to test for a possible quenching effect of the candidate substance on tryptophan fluorescence. This quenching of tryptophan fluorescence is still measurable at 440 nm and influences the intensity and reliability of the FRET signal when using MANT-uracil. For that reason we measured in parallel the emission at 440 nm for the following three reaction mixtures containing:

1. CRBN homologue mutant W36/59F from *M. gryphiswaldense* (final 3μM) and several concentrations of candidate substance in assay buffer;
2. several concentrations of candidate substance in assay buffer,
3. CRBN homologue mutant W36/59F from *M. gryphiswaldense* (final 3μM), MANT-uracil (final 3 μM) and several concentrations of candidate substance in assay buffer. If the plot of the fluorescence emission data obtained from mixture 1 against the candidate substance concentration shows the characteristics of a binding curve, the substance obviously quenches the fluorescence of the tryptophan residues in the binding pocket. Accordingly, the FRET data measured from mixture 3 resemble in this case a summation of tryptophan quenching and the FRET effect and are therefore not suitable for calculating $K_d$ values.

This was the case for the following substances tested so far: uridine, deoxyuridine, uracil, glutarimide, thalidomide, thymidine and 2-pyrrolidone. For these substances we used the quenching effect to determine the $K_d$ values for the ligands. As it is shown for uridine in Fig. 8, we titrated increasing concentrations of uridine in 100 μl of a 3 μM protein solution in assay buffer and the according references as described above (example 5). The measurements were performed as described. Due to the decreasing signal intensities with increasing ligand concentrations, the signal data analysis was performed using reciprocal values of fluorescence emission (measured at 440 or preferred at 370 nm).

The obtained $K_d$ values are 5.8 μM for thalidomide, 7 μm for deoxyuridine, 14.5 μM for uridine, 136 μM for uracil, 248 μM for 2-pyrrolidone, 330 μm for glutarimide and 431 μM for thymidine. In contrast no binding could be detected for UMP and cytidine at concentrations up to 1 mM.

If the plot of the fluorescence emission data obtained from mixture 1 against the candidate substance concentration does not resemble the characteristics of a binding curve, the inhibitory constant $K_i$ can be calculated from the FRET data measured from mixture 3 using the formula:

$$K_i = \frac{IC_{50}}{1 + \frac{[S]}{K_d}} .$$

The $K_d$-value corresponds to 1.13 μM as calculated for MANT-uracil binding, the protein concentration [S] is 3 μM and the according $IC_{50}$ value can be determined as described for thalidomide binding in example 6.

**Example 8: Assay using CRBN-homologue from *C. elegans***

The determination of the binding constant $K_d$ of MANT-uracil and the $IC_{50}$ value of thalidomide in the assay with the CRBN-homologue from *C. elegans* of SEQ ID No. 9 was performed as described for the CRBN-homologue of *M. gryphiswaldense* in examples 5 and 6 . Due to the arginine in the storage buffer of the C. elegans protein, the assay buffer contained additionally a final concentration of 10 mM arginine.

The estimated binding constant for MANT-uracil is $K_d$ = 0.348 ± 0.1 μM.

The calculated $IC_{50}$ value for the inhibition of MANT-uracil binding to the *C. elegans* CRBN-homologue by

thalidomide in this assay is calculated with 14.16 μM.

**References**

Emsley, P. and Cowtan, K. (2004) Coot: model-building tools for molecular graphics, Acta Crystallogr. D Biol. Crystallogr, 60, 2126-2132.

Kabsch, W. (1993) Automatic processing of rotation diffraction data from crystals of initially unknown symmetry and cell constants. J. Appl. Crystallogr, 26, 795-800.

Murshudov, G.N., Vagin, A.A., Lebedev, A., Wilson, K.S. and Dodson, E.J. (1999) Efficient anisotropic refinement of macromolecular structures using FFT, Acta Crystallogr. D Biol. Crystallogr, 55, 247-255.

Perrakis, A., Morris, R. and Lamzin, V.S. (1999) Automated protein model building combined with iterative structure refinement. Nature Struct. Biol, 6, 458-463.

Sheldrick, G.M. (2008). A short history of SHELX. Acta Cryst. A, 64, 112-122.

## Claims

1. Method of identifying substances which bind to a thalidomide binding peptide based on determining alteration of tryptophan fluorescence within the binding pocket of the thalidomide binding peptide.

2. Method of identifying substances which bind to a thalidomide binding peptide according to claim 1 comprising the following steps:

   a) providing a thalidomide binding peptide
   b) providing a compound binding to said thalidomide binding peptide interacting with the tryptophan fluorescence of the thalidomide binding peptide
   c) providing a candidate substance to be tested
   d) forming a complex of the thalidomide binding peptide with the compound of step b)
   e) forming a reaction mixture by contacting the candidate substance with the complex of step d)
   f) incubating the reaction mixture under conditions sufficient to allow the candidate substance to replace the compound of step b) in the complex, in case the candidate substance is able to bind to the thalidomide binding peptide,
   g) determining if the compound of step b) has been replaced in the complex by the candidate substance.

3. Method according to claim 2, wherein the compound of step b) has an excitation frequency between 310 and 425 nm.

4. Method according to claim 3, wherein step g) comprises measuring the emission of the reaction mixture at the emission frequency of the compound of step b).

5. Method according to claim 1, comprising the following steps:

   A) providing a thalidomide binding peptide
   B) providing a candidate substance to be tested
   C) forming a reaction mixture by contacting the candidate substance with the thalidomide binding peptide
   D) incubating the reaction mixture under conditions sufficient to allow the candidate substance to bind to the thalidomide binding peptide, in case the candidate substance is able to bind to the thalidomide binding peptide,
   E) determining if the candidate substance forms a complex with the thalidomide binding peptide using alteration in tryptophan fluorescence within the binding pocket of the thalidomide binding peptide.

6. Method according to claim 5, comprising further the following steps F) - H):

   F) adding a compound binding to said thalidomide binding peptide and having an excitation frequency between 310 and 425 nm.
   G) incubating the reaction mixture under conditions sufficient to allow the compound of step F) to replace the candidate substance in the complex, and
   H) determining if the candidate substance has been replaced in the complex compound of step F).

7. Method according to any one of claims 1 - 6, wherein replacement is indicative for a teratogenic potential of the candidate substance.

8. Method according to any one of claims 1 - 7, wherein the thalidomide binding peptide is chosen from cereblon, a cereblon homologue, the thalidomide-binding domain of cereblon, a peptide homologous to the thalidomide binding domain of cereblon having the amino acid sequence of SEQ ID No. 7 or a peptide homologous to the thalidomide binding domain of the cereblon homologue of *M. gryphiswaldense* comprising the amino acid sequence of SEQ ID No. 8, whereas two sequences are regarded homologous when their alignment yields expect values (E-values) better than 1.0e-5 as determined by *Basic Local Alignment Search Tool.*

9. A kit for identifying test compounds having a teratogenic potential containing

   a) a thalidomide binding peptide
   b) compound having an emission between 310 - 425 nm and a subunit binding to said thalidomide binding peptide.

10. The fluorescent compound 1-(N-2-MANT-imidoethyl)-uracil.

**11.** A mutant cereblon homologue of *M. gryphiswaldense* with an amino acid sequence having at least a modification corresponding to amino acid residues 36 and 59 of SEQ ID No. 1.

**12.** Nucleic acid molecule comprising a sequence as set forth in SEQ ID No. 3 that encodes a cereblon homologue of *M. gryphiswaldense* or a nucleic acid having at least 35% sequence identity to SEQ ID No. 4, wherein the nucleic acid sequence is codon-optimized for high level expression in *Escherichia coli.*

**13.** Co-crystal form of cereblon homologue of *M. gryphiswaldense* with a ligand characterized as having a space group of $P2_12_12_1$, three monomers of cereblon homologue of *M. gryphiswaldense* in the asymmetric unit and unit cell dimension of $a_1$ = 55 - 58 Å, $b_1$ = 58-61 Å, $c_1$ = 85-90 Å, $\alpha_1 = \beta_1 = \gamma$ = 90 °; or having a space group of $P3_221$, one monomer of cereblon homologue of *M. gryphiswaldense* in the asymmetric unit and unit cell dimension of $a_2 = b_2$ = 50 - 53 Å, $c_2$ = 82 - 87 Å, $\alpha_2 = \beta_2$ = 90 ° and $\gamma$ = 120 °; or having a space group of $P6_122$, four monomers of cereblon homologue of *M. gryphiswaldense* in the asymmetric unit and unit cell dimension of $a_3 = b_3$ = 135 - 140 Å, $c_3$ = 151 - 160Å, $\alpha_3 = \beta_3$ = 90 ° and $\gamma$ = 120°.

**14.** Method for co-crystallizing cereblon homologue of *M. gryphiswaldense* or a peptide comprising at least the binding pocket of the cereblon homologue of *M. gryphiswaldense* and a ligand comprising

i) providing a buffered, aqueous solution containing cereblon homologue of *M. gryphiswaldense*;
ii) adding a ligand to the aqueous solution of cereblon homologue of *M. gryphiswaldense*
iii) growing co-crystal forms by mixing the mixture of step ii) with a reservoir solution of pH 3,5 - 8 comprising between 5 % and 30 %(w/v) of a precipitant and equilibrated against reservoir solution.

**15.** Method for identifying a compound that interacts with cereblon homologue of *M. gryphiswaldense* or a peptide comprising at least the binding pocket of the cereblon homologue of *M. gryphiswaldense,* comprising the step of

I) providing a co-crystal form according to claim 12 or 13.
II) providing a soaking solution of 1 - 10 mM of a candidate substance in a solution
III) incubating the crystals of step I) with the soaking solution of step II) for a time sufficient that individual monomers in the unit cells of the co-crystal exchange the ligand by the test substance.

**Figure 1**

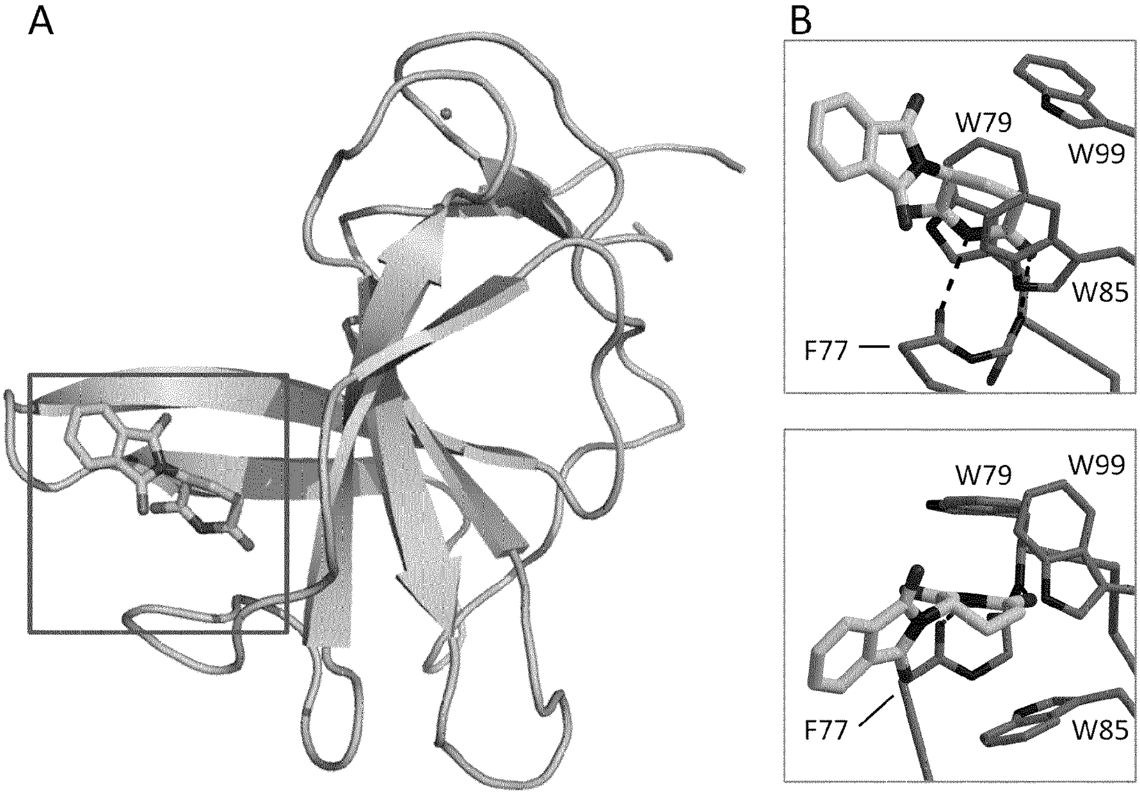

## Figure 2

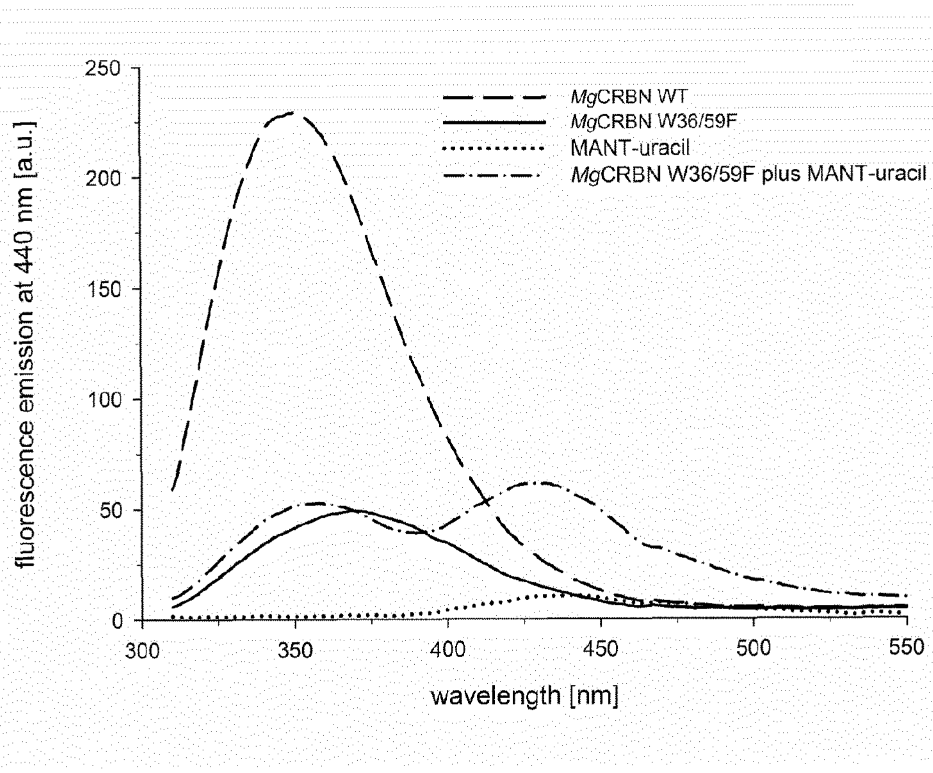


## Figure 3

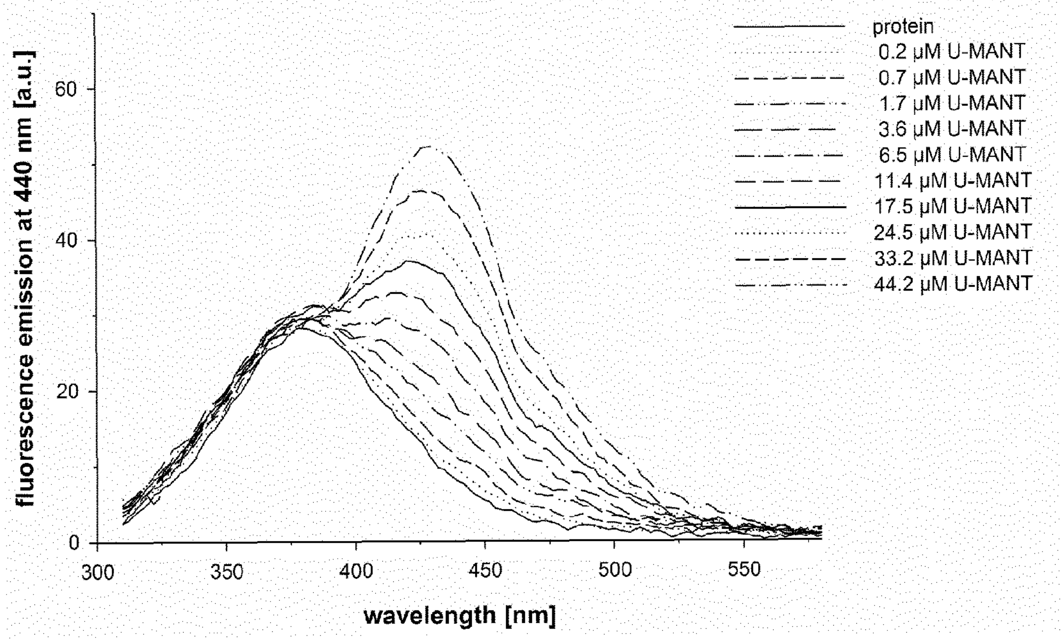

Figure 4 A

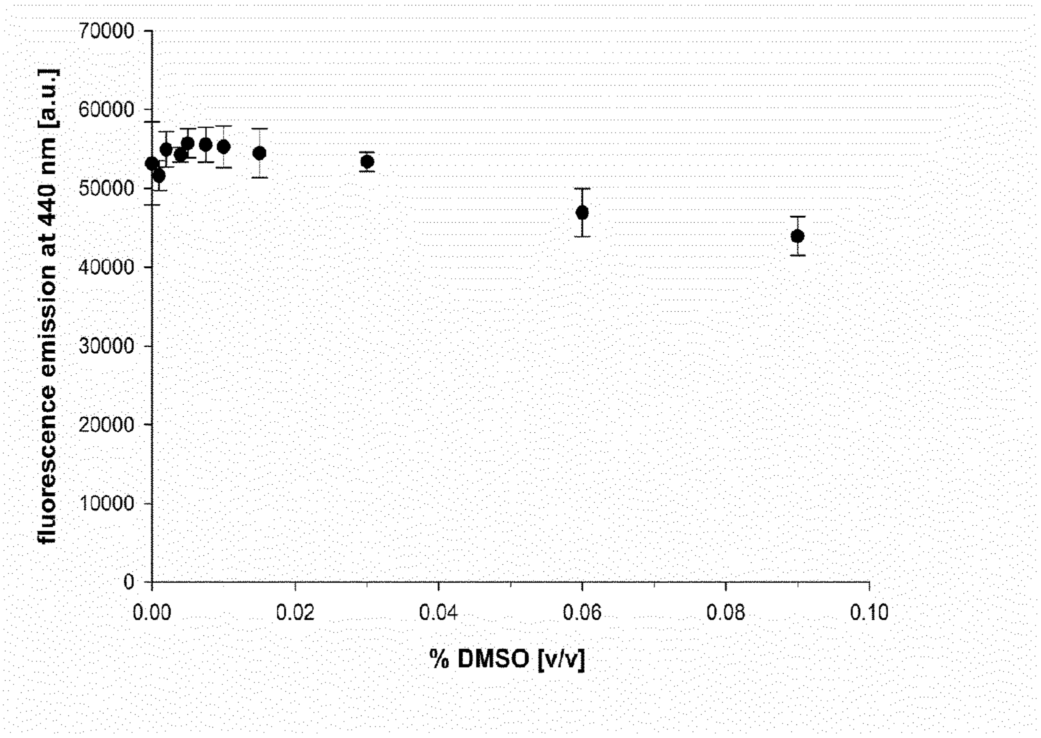


Figure 4 B

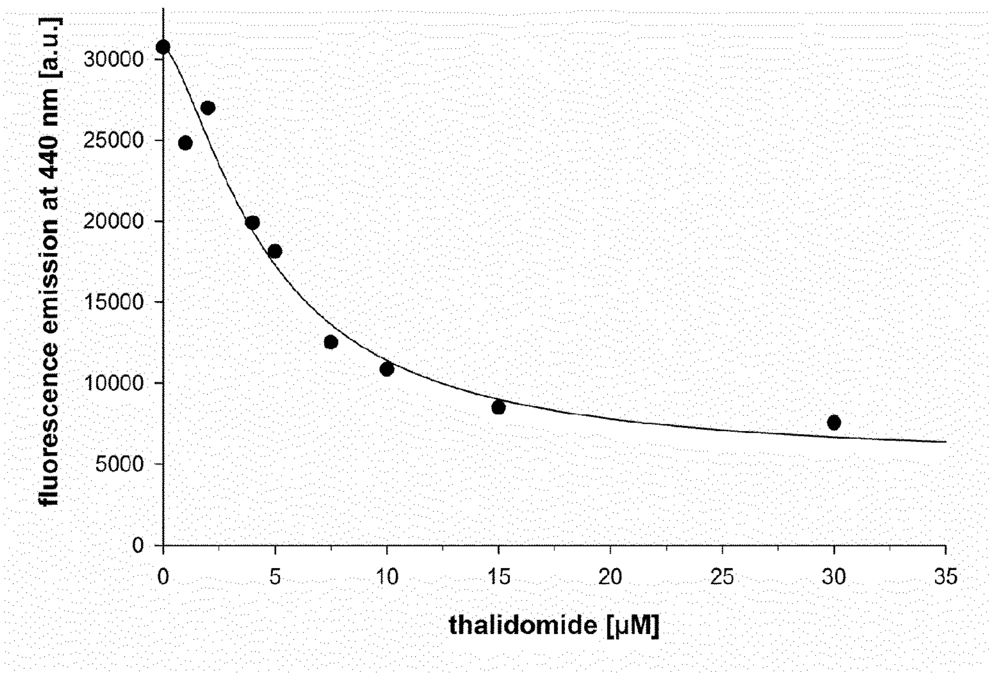

Figure 5

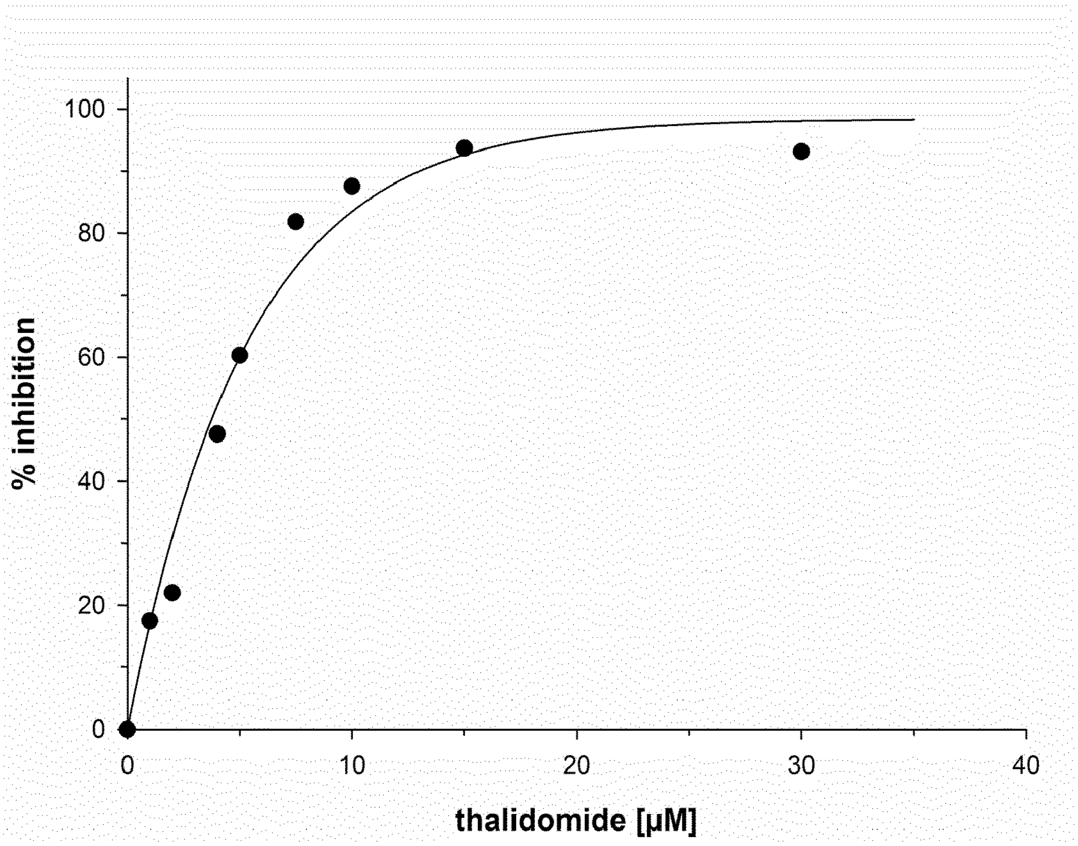

Figure 6 A

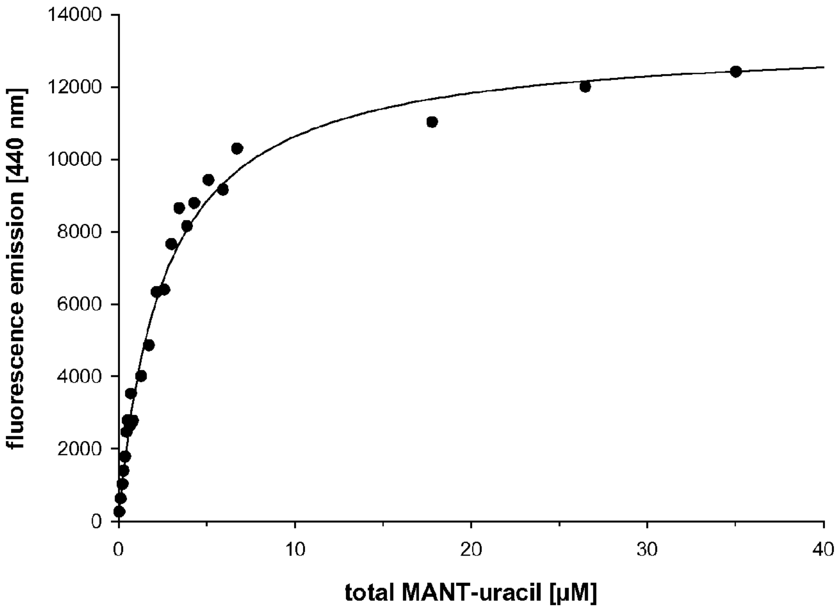


Figure 6 B

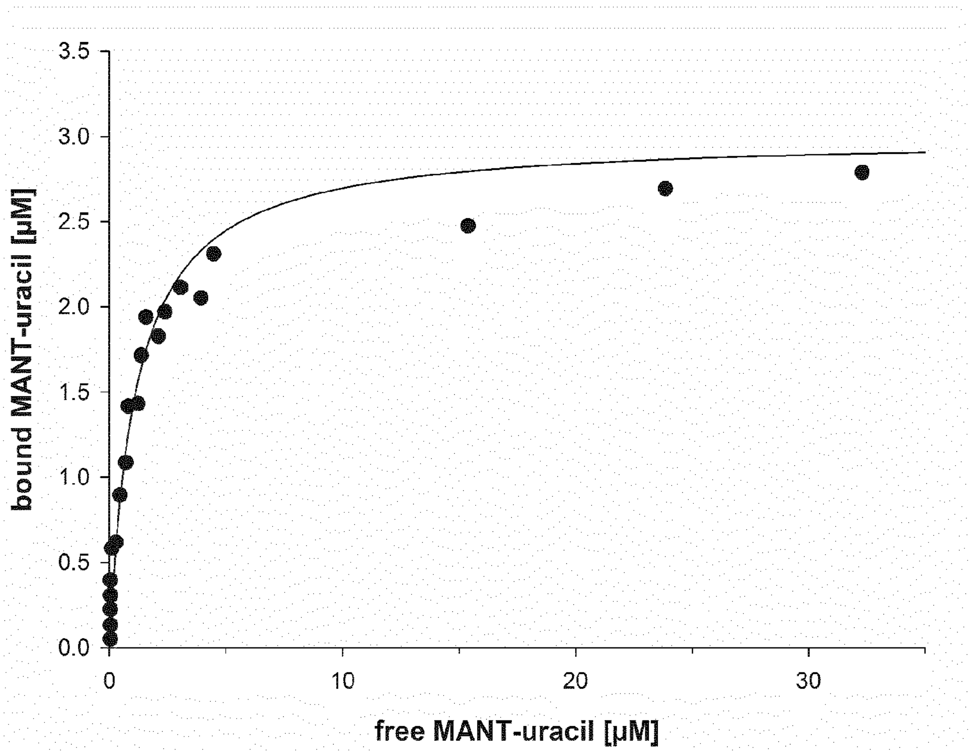

Figure 7

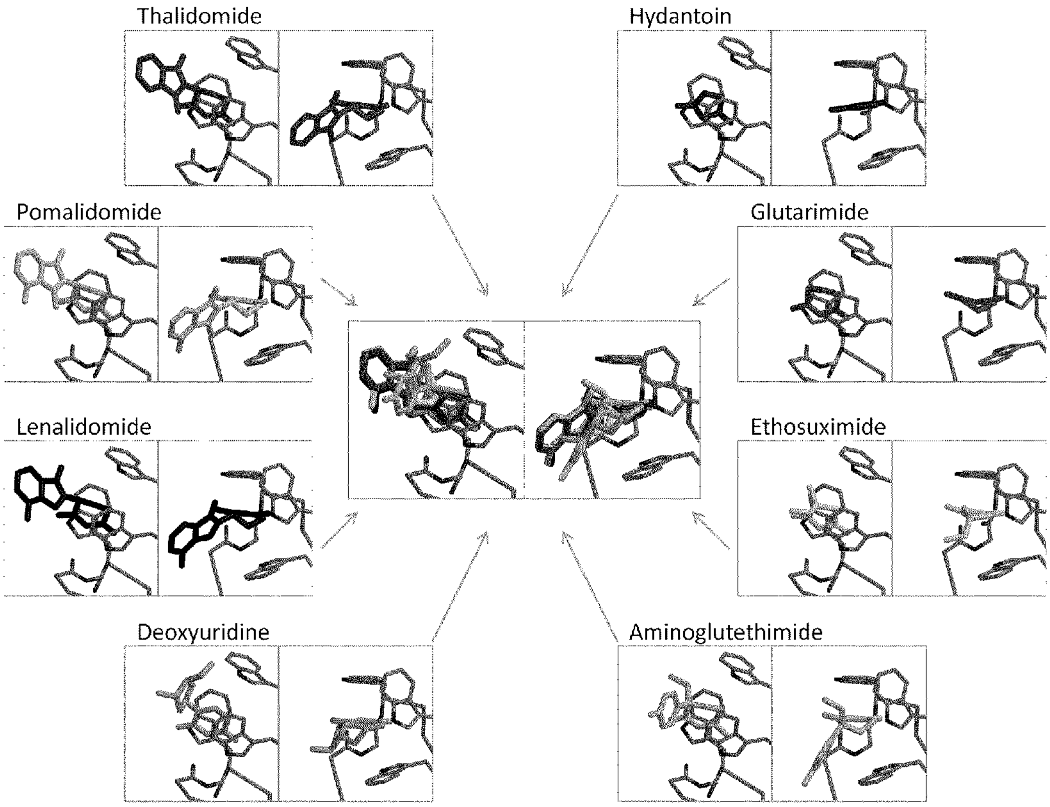
Thalidomide
Hydantoin
Pomalidomide
Glutarimide
Lenalidomide
Ethosuximide
Deoxyuridine
Aminoglutethimide

Figure 8

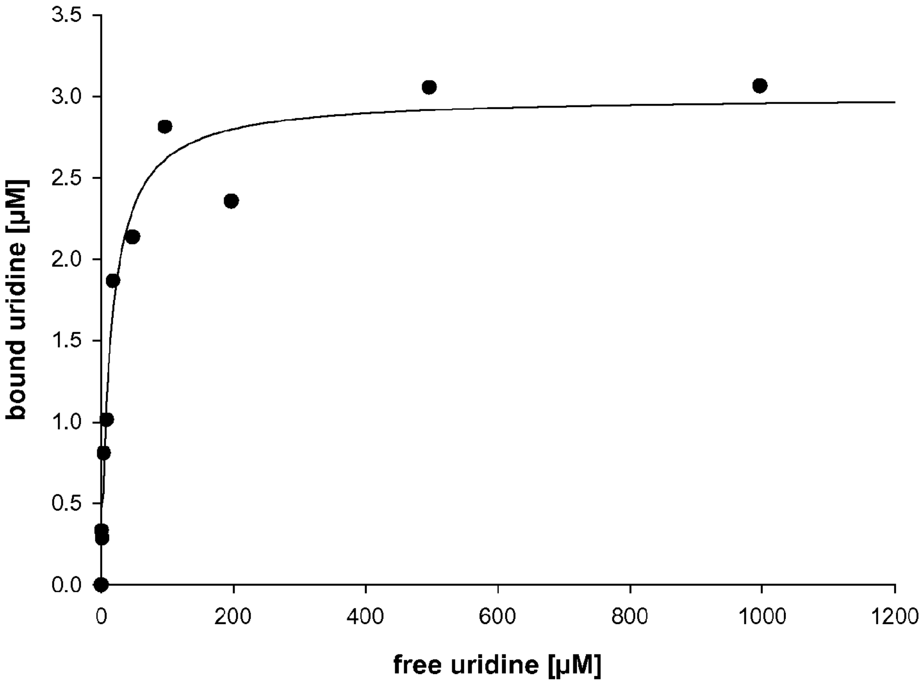


Figure 9A

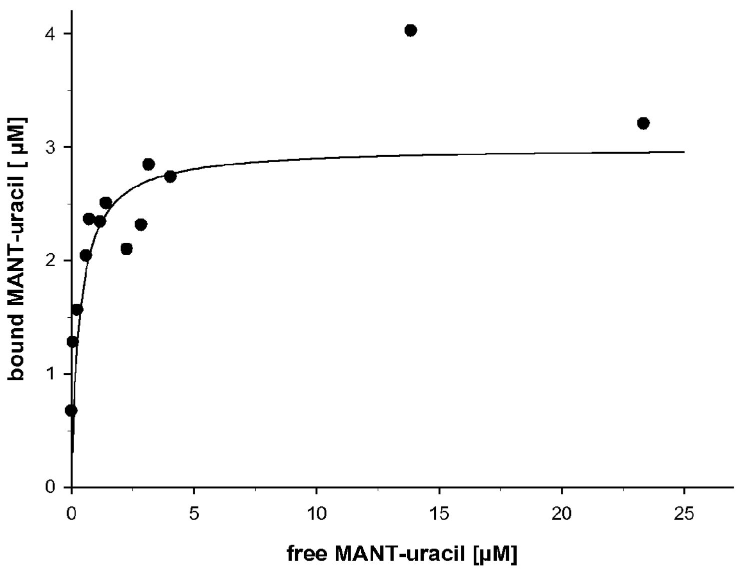

**Figure 9B**

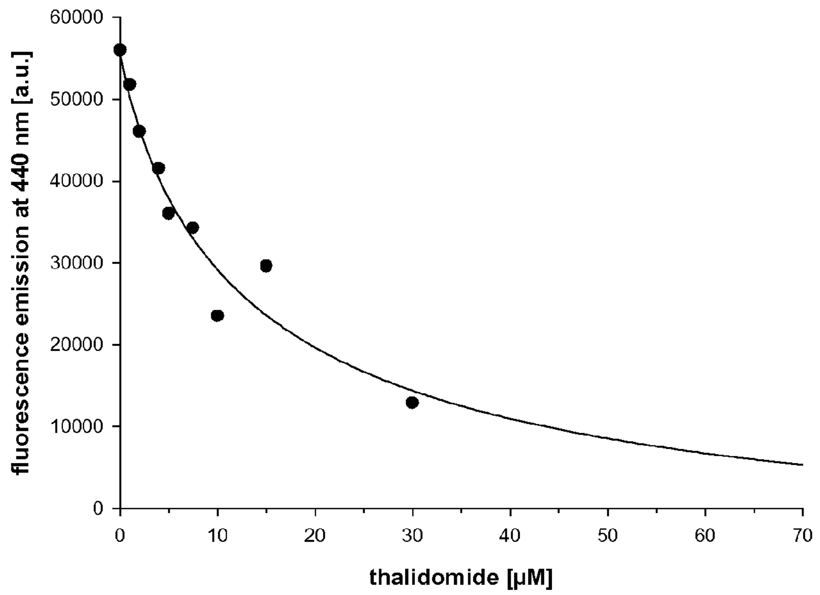


**Figure 10**

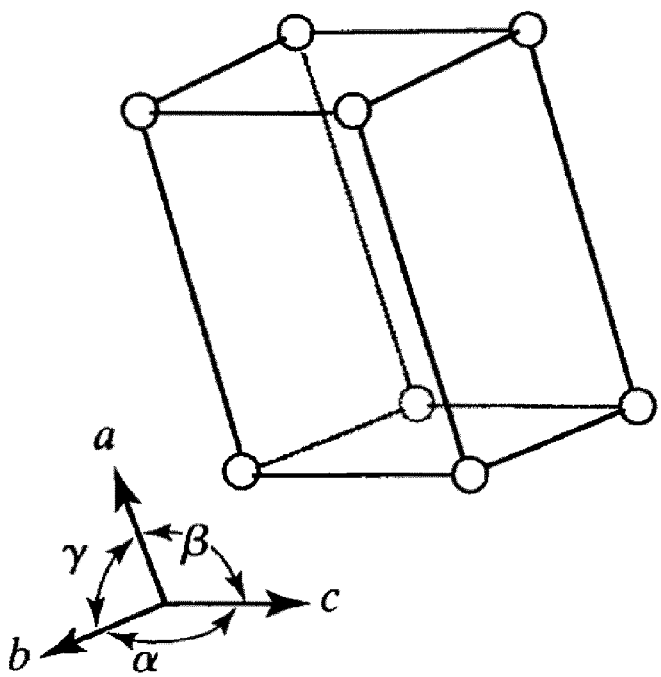

Europäisches Patentamt
European Patent Office
Office européen des brevets

# EUROPEAN SEARCH REPORT

Application Number
EP 13 15 1538

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | T. ITO ET AL: "Identification of a Primary Target of Thalidomide Teratogenicity", SCIENCE, vol. 327, no. 5971, 12 March 2010 (2010-03-12), pages 1345-1350, XP055062167, ISSN: 0036-8075, DOI: 10.1126/science.1177319 | 1,5,7,8 | INV. G01N33/68 |
| A | * page 1345, column 3 - page 1346, column 1, paragraph 1; figures 1,3 * | 2-4,6,9 | |
| | ----- | | |
| Y | US 2012/192297 A1 (HANDA HIROSHI [JP] ET AL) 26 July 2012 (2012-07-26) | 1,5,7,8 | |
| A | * abstract * & EP 2 492 686 A1 (TOKYO INST TECH [JP]; FUJIMOTO SEIYAKU KK [JP]) 29 August 2012 (2012-08-29) | 2-4,6,9 | |
| | ----- | | |
| Y | POLLARD THOMAS D: "A Guide to Simple and Informative Binding Assays", MOLECULAR BIOLOGY OF THE CELL, vol. 21, no. 23, December 2010 (2010-12), pages 4061-4067, XP055063698, ISSN: 1059-1524 | 1,5,7,8 | TECHNICAL FIELDS SEARCHED (IPC) |
| | | | G01N |
| A | * abstract * * page 4063, column 2, last paragraph - page 4064, column 1, paragraph 1; figure 2 * | 2-4,6,9 | |
| | ----- | | |

~~The present search report has been drawn up for all claims~~

1

| Place of search | Date of completion of the search | Examiner |
|---|---|---|
| The Hague | 22 May 2013 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 13 15 1538

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 13 15 1538

The Search Division considers that the present European patent application does not comply with the requirements of unity of invention and relates to several inventions or groups of inventions, namely:

1. claims: 1-9

   Method of identifying substances which bind to thalidomide binding peptide and products involved

   ---

2. claim: 10

   1-(N-2-MANT-imidoethyl)-uracil

   ---

3. claims: 11, 12

   A mutant cereblon homologue of M. gryphiswaldense

   ---

4. claims: 13-15

   Co-crystal form of cereblon homologue with a ligand and method of producing it and its use.

   ---

## ANNEX TO THE EUROPEAN SEARCH REPORT ON EUROPEAN PATENT APPLICATION NO. EP 13 15 1538

This annex lists the patent family members relating to the patent documents cited in the above-mentioned European search report. The members are as contained in the European Patent Office EDP file on
The European Patent Office is in no way liable for these particulars which are merely given for the purpose of information.

22-05-2013

| Patent document cited in search report | Publication date | Patent family member(s) | Publication date |
|---|---|---|---|
| US 2012192297 A1 | 26-07-2012 | AU 2010309035 A1 | 12-04-2012 |
| | | CA 2774871 A1 | 28-04-2011 |
| | | CN 102576023 A | 11-07-2012 |
| | | EP 2492686 A1 | 29-08-2012 |
| | | KR 20120093852 A | 23-08-2012 |
| | | US 2012192297 A1 | 26-07-2012 |
| | | WO 2011049043 A1 | 28-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2492686 A1


### Non-patent literature cited in the description


- **ITO et al.** *Science,* vol. 327, 1345-1350
- Sax's Dangerous Properties of Industrial Materials
- **EMSLEY, P. ; COWTAN, K.** Coot: model-building tools for molecular graphics. *Acta Crystallogr. D Biol. Crystallogr,* 2004, vol. 60, 2126-2132
- **KABSCH, W.** Automatic processing of rotation diffraction data from crystals of initially unknown symmetry and cell constants. *J. Appl. Crystallogr,* 1993, vol. 26, 795-800
- **MURSHUDOV, G.N. ; VAGIN, A.A. ; LEBEDEV, A. ; WILSON, K.S. ; DODSON, E.J.** Efficient anisotropic refinement of macromolecular structures using FFT. *Acta Crystallogr. D Biol. Crystallogr,* 1999, vol. 55, 247-255
- **PERRAKIS, A. ; MORRIS, R. ; LAMZIN, V.S.** Automated protein model building combined with iterative structure refinement. *Nature Struct. Biol,* 1999, vol. 6, 458-463
- **SHELDRICK, G.M.** A short history of SHELX. *Acta Cryst. A,* 2008, vol. 64, 112-122